# EUROPÄISCHE PATENTSCHRIFT

(11) **EP 3 160 934 B1**
(45) Veröffentlichungstag und Bekanntmachung des Hinweises auf die Patenterteilung: **26.01.2022**
(21) Anmeldenummer: 15729526.2
(22) Anmeldetag: 22.06.2015
(51) Int. Cl.: C07C 209/78

(54) **VERFAHREN ZUR HERSTELLUNG VON DI- UND POLYAMINEN DER DIPHENYLMETHANREIHE**
METHOD FOR PRODUCING DI- AND POLYAMINES OF THE DIPHENYLMETHANE SERIES
PROCÉDÉ DESTINÉ À LA FABRICATION DE DI- ET POLYAMINES DE LA SÉRIE DIPHÉNYLMÉTHANE

(30) Priorität: 24.06.2014 EP 14173580
(43) Veröffentlichungstag der Anmeldung: 03.05.2017
(73) Patentinhaber: Covestro Intellectual Property GmbH & Co. KG, 51373 Leverkusen (DE)
(72) Erfinder: KNAUF, Thomas, 41542 Dormagen (DE); WERSHOFEN, Stefan, 41065 Mönchengladbach (DE); GRUNER, Klaus-Gerd, 47239 Duisburg (DE); HARTJES, Volker, 47239 Duisburg (DE)
(74) Vertreter: Levpat
(86) Internationale Anmeldenummer: PCT/EP2015/063934
(87) Internationale Veröffentlichungsnummer: WO 2015/197527

(56) Entgegenhaltungen:
- EP-A1- 0 283 757
- EP-A1- 1 616 890
- EP-A1- 2 039 676

## Beschreibung

Die vorliegende Erfindung betrifft ein Verfahren zum Betrieb einer Anlage zur Herstellung von Di- und Polyaminen der Diphenylmethanreihe (MDA). Die Erfindung ermöglicht es, Produktionsstillstände beim Betreiben des MDA-Prozesses hinsichtlich Zeitaufwand und gegebenenfalls auch hinsichtlich Energie- und Materialverbrauch durch eine sog. Kreislauffahrweise einzelner Anlagenteile zu optimieren. Während einer Unterbrechung des Verfahrens bzw. Unterbrechung des Betriebs einzelner Anlagenteile findet kein Eintrag von Formaldehyd in die Reaktion statt, und die nicht von einer Revisions-, Reparatur- oder Reinigungsmaßnahme betroffenen Anlagenteile werden in sog. Kreislauffahrweise betrieben. Dadurch wird unter anderem erreicht, dass nur das betroffene Anlagenteil für die Zeit der Maßnahme stillgelegt werden muss, was vorteilhaft hinsichtlich Produktivität und Wirtschaftlichkeit des Verfahrens sowie der Qualität der hergestellten Produkte sein kann.

Die kontinuierliche oder teilweise diskontinuierliche Herstellung von MDA ist z. B. in EP 1 616 890 A1, US-A-5286760, EP-A-451442 und WO-A-99/40059 offenbart. Die saure Kondensation von aromatischen Aminen und Formaldehyd zu Di- und Polyaminen der Diphenylmethanreihe läuft in mehreren Reaktionsschritten ab.

Beim Aminalverfahren wird zunächst in Abwesenheit eines sauren Katalysators Formaldehyd mit Anilin zu sogenanntem Aminal kondensiert, wobei Wasser abgespalten wird. Danach erfolgt die Umlagerung zum MDA säurekatalysiert in einem ersten Schritt zu para- bzw. ortho-Aminobenzylanilin. Die Aminobenzylaniline lagern in einem zweiten Schritt zum MDA um. Hauptprodukte der säurekatalysierten Reaktion von Anilin und Formaldehyd sind das Diamin 4,4'-MDA, seine Stellungsisomere 2,4'-MDA und 2,2'-MDA sowie höhere Homologe.

Beim Neutralisationsverfahren werden in Anwesenheit eines sauren Katalysators Anilin und Formaldehyd direkt zu Aminobenzylanilinen umgesetzt, die anschließend weiter zu den zweikernigen MDA-Isomeren und höherkernigen MDA-Homologen reagieren.

Unabhängig von der Verfahrensvariante zur Herstellung des sauren Reaktionsgemisches wird dessen Aufarbeitung gemäß dem Stand der Technik durch Neutralisation mit einer Base eingeleitet. Die Neutralisation erfolgt üblicherweise bei Temperaturen von beispielsweise 90 °C bis 100 °C ohne Zusatz weiterer Substanzen (H.J. Twitchett, Chem. Soc. Rev. 3(2), 223 (1974)). Sie kann aber auch auf einem anderen Temperaturniveau erfolgen, um z. B. den Abbau von störenden Nebenprodukten zu beschleunigen. Hydroxide der Alkali- und Erdalkalielemente sind als Basen geeignet. Vorzugsweise kommt wässrige NaOH zum Einsatz.

Im Anschluss an die Neutralisation wird in einem Trennbehälter die organische von der wässrigen Phase getrennt. Die nach Abtrennung der wässrigen Phase verbleibende, Roh-MDA enthaltende organische Phase wird weiteren Aufarbeitungsschritten unterzogen, wie z. B. einer Wäsche mit Wasser (Basenwäsche), um Restsalze aus dem Roh-MDA zu waschen. Abschließend wird das so gereinigte Roh-MDA von überschüssigem Anilin, Wasser und anderen im Gemisch vorhandenen Stoffen (z. B. weiteren Lösungsmitteln) durch geeignete Verfahren wie z. B. Destillation, Extraktion oder Kristallisation befreit. Die nach dem Stand der Technik übliche Aufarbeitung ist beispielsweise offenbart in EP 1 652 835 A1, Seite 3, Zeile 58 bis Seite 4, Zeile 13 oder EP 2 103 595 A1, Seite 7, Zeile 21 bis 37.

EP 1 616 890 A1 lehrt, dass Anilin und Formaldehyd zunächst in Abwesenheit des sauren Katalysators zu Aminal umgesetzt werden, und das Aminal anschließend mit saurem Katalysator versetzt wird, und es bei Temperaturen von 20 °C bis 100 °C und bei Wassergehalten des so erhaltenen sauren Reaktionsgemisches von 0 bis 20 Gewichtsprozent weiter umgesetzt wird. Insbesondere wird nach der Kondensation von Formaldehyd und Anilin zum Aminal zunächst das Wasser zumindest teilweise aus dem Aminal entfernt, wobei man einen Wassergehalt von 0 bis 5 Gewichtsprozent im Aminal einstellt, und anschließend das Aminal mit saurem Katalysator versetzt, und es bei Temperaturen von 20 °C bis 100 °C und bei Wassergehalten des so erhaltenen sauren Reaktionsgemisches von 0 bis 20 Gewichtsprozent weiter umsetzt. So können Gemische der Di- und Polyamine der Diphenylmethanreihe bei Protonierungsgraden von < 15 %, bevorzugt 4 % bis 14 %, besonders bevorzugt 5 % bis 13 %, hergestellt werden. Als Protonierungsgrad wird dabei bei einprotonigen sauren Katalysatoren (wie Salzsäure) das molare Verhältnis aus der Menge an eingesetztem sauren Katalysator und der im Reaktionsgemisch vorhandenen molaren Menge an Aminfunktionen bezeichnet. Die Patentanmeldung geht in keiner Weise auf die Vorgehensweise während der Außerbetriebnahme einzelner Anlagenteile einer großtechnischen Produktionsanlage ein. Die enthaltenen Beispiele sind Laborexperimente. Insbesondere lehrt diese Patentanmeldung nicht, dass zur Außerbetriebnahme lediglich einzelner Anlagenteile nicht notwendigerweise die Gesamtanlage *vollständig heruntergefahren* werden muss.

Die Patentanmeldung EP 2 039 676 A1 befasst sich mit der Herstellung von MDA mit dem Schwerpunkt auf Neutralisation und Wäsche. Beschrieben wird eine Verfahrensweise zur Optimierung der Phasentrennung in der Neutralisation (Schritt c) und/oder der Wäsche (Schritt e2). Ein Teil der wässrigen Phase aus der Phasentrennung der Wäsche e2) oder der Aminabtrennung aus dem Abwasser e3) wird zurück in die Neutralisation oder die Wäsche gefahren. Mit anderen Worten: beschrieben wird die Verschaltung einer MDA-Anlage *im kontinuierlichen Regelbetrieb.* Eine Vorgehensweise bei einem Stillstand der MDA-Anlage, bei dem einzelne Anlagenteile außer Betrieb genommen werden und andere Analgenteile in Kreislauffahrweise betrieben werden, ist der Patentanmeldung nicht zu entnehmen.

EP 0 283 757 A1 befasst sich ebenfalls mit der Herstellung von MDA. Das beschriebene Verfahren ist gekennzeichnet durch die Zufuhr anilinfreien MDA's zu durch Kondensation von Anilin und Formaldehyd gebildeten Aminobenzylaminen vor deren durch Wärme induzierter Umlagerungsreaktion. In Beispiel 2 wird eine Verfahrensweise beschrieben, bei der ein geringer Teil des gebildeten MDA in die Umlagerungsreaktion zurückgeführt wird (vgl. auch Patentansprüche 6 und 8). Mit anderen Worten: beschrieben wird die Verschaltung einer MDA-Anlage *im kontinuierlichen Regelbetrieb.* Details zur Vorgehensweise bei Außerbetriebnahme einzelner Anlagenteile sind der Patentanmeldung nicht zu entnehmen.

WO-A-99/40059 lehrt, dass man zur Herstellung von Metyhlendi(phenylamin) durch Umsetzung von Anilin mit Formaldehyd in Gegenwart saurer Katalysatoren in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50 % der gesamt einzuspeisenden Formaldehydmenge die Reaktionsmischung auf eine Temperatur von größer 75 °C temperiert. Die Zugabe bis zu einer Menge von mindestens 50 % der gesamt einzuspeisenden Formaldehydmenge erfolgt bei einer Temperatur des Reaktionsgemisches im Kreislauf von 20 °C bis 75 °C.

Die Güte eines Verfahrens zur Herstellung von MDA ist einerseits definiert durch den Gehalt des Produktes an unerwünschten Nebenprodukten der Reaktion. Andererseits ist die Güte eines Verfahrens dadurch definiert, dass der gesamte Prozess von Anfahren, normaler Produktion bis zum Abfahren des Prozesses ohne technischen Produktionsausfall oder Problemen, die zu einem Eingriff in den Prozess nötigen, betrieben werden kann, und dass es nicht zu Verlusten an Einsatzstoffen, Zwischenprodukten oder an Endprodukt kommt.

Solche Probleme können z. B. beim Anfahren bzw. Abfahren der Aminalreaktion auftreten. Derartige Probleme können z. B. sein, dass es zur Entstehung von hochmolekularen Feststoffen kommt, die zu Anbackungen und Verblockungen an der Ausrüstung (Aminalkessel, -kühler und -abscheider und Leitungen) führen. Nachteilig ist des Weiteren, dass bei notwendigen Wartungs-, Reparatur- und Reinigungsarbeiten an oder in einem Reaktor oder einem sonstigen Anlagenteil regelmäßig immer alle Anlagenteile abgeschaltet werden müssen, da die Verfahrensschritte aufeinander aufbauen und somit immer sukzessive erfolgen. Dadurch muss die gesamte Anlage geleert werden, was zu einem erheblichen Materialausschuss führt. Des Weiteren muss Energie aufgewendet werden, um Reaktoren und Anlagenteile wieder auf die jeweiligen Betriebstemperaturen zu bringen. Solche Produktionsstillstände für Anlagenrevisionen, Reparatur- und Reinigungsmaßnahmen oder auftretende Rohstoff- oder Hilfsstoffmängel, geplant oder ungeplant, sind daher immer wiederkehrende Anlagenzustände, die einen erheblichen Einfluss auf das wirtschaftliche Betreiben einer kontinuierlich arbeitenden Anlage bzw. eines kontinuierlich arbeitenden Verfahrens haben.

Den beschriebenen Verfahren des Standes der Technik gelingt es zwar, mit hoher Ausbeute MDA herzustellen, ohne dass es bei den Endprodukten zu einer Qualitätseinbuße kommt, jedoch werden nur Verfahren beschrieben, die sich im Normalbetrieb befinden. Prozesse oder Anlagen, die Produktionsstillstände für Anlagenrevisionen, Reparaturmaßnahmen oder beispielsweise Rohstoff- oder Hilfsstoffmangel ausreichend berücksichtigen, sind bisher nicht im Stand der Technik beschrieben worden. Wünschenswert wären also Verfahren und Anlagen zur Herstellung von Di- und Polyaminen der Diphenylmethanreihe, bei denen es möglich ist, Produktionsstillstände beim Betreiben des MDA-Prozesses hinsichtlich Zeitaufwand und gegebenenfalls auch hinsichtlich Energie- und Materialverbrauch zu optimieren. Diese würden in einem nicht unerheblichen Umfang zu einer Verbesserung der Produktivität und somit der Wirtschaftlichkeit eines kontinuierlich arbeitenden MDA-Herstellungsprozesses und den entsprechenden Anlagen dafür führen.

Die vorliegende Erfindung stellt daher Folgendes bereit:
Ein **Verfahren zum Betrieb einer Anlage zur Herstellung von Di- und Polyaminen der Diphenylmethanreihe** (**MDA**), wobei das Verfahren einen Produktionsstillstand umfasst und die Anlage die folgenden Anlagenteile umfasst:
IA) einen Reaktor mit integrierter Phasentrenneinrichtung oder einen Reaktor und einen separaten Phasentrennapparat zur Reaktion von Anilin und Formaldehyd in Abwesenheit eines sauren Katalysators unter Bildung eines Aminals und anschließender Trennung des erhaltenen Reaktionsgemisches in eine wässrige und eine organische, das Aminal enthaltenden Phase und
IIA) einen Reaktor zur Reaktion der im Reaktor IA) erhaltenen organischen, das Aminal enthaltenden Phase mit Säure,
   oder
IB) einen Reaktor zur Reaktion von Anilin mit Säure und
IIB) einen Reaktor zur Reaktion des im Reaktor IB) erhaltenen Reaktionsgemisches mit Formaldehyd;
   und
III) einen Reaktor zur Neutralisation des Reaktionsgemisches aus IIA) oder IIB);
IV) einen Trennbehälter zum Auftrennen des neutralisierten Reaktionsgemisches aus III) in eine organische Phase, umfassend Di- und Polyamine der Diphenylmethanreihe und eine wässrige Phase;
V) einen Waschbehälter zum Waschen der organischen Phase aus IV) mit Waschflüssigkeit;
VI) einen Trennbehälter zum Auftrennen des Gemisches aus V) in eine organische Phase, umfassend Di- und Polyamine der Diphenylmethanreihe und eine wässrige Phase;
VII) eine Destillationseinrichtung zum Destillieren der organischen Phase aus VI) unter Erhalt von Di- und/oder Polyaminen der Diphenylmethanreihe und eines Wasser und Anilin enthaltenden Stroms;
   sowie bevorzugt
VIII) eine Abwasseraufarbeitungseinrichtung zur Aufarbeitung der wässrigen Phase aus IA) und/oder der wässrigen Phase aus IV) und/oder der wässrigen Phase aus VI) und/oder des Wasser und Anilin enthaltenden Stroms aus VII), bevorzugt umfassend einen Abwassersammelbehälter, Abwassererhitzer und Anilintrennbehälter;
wobei zur Durchführung des Produktionsstillstandes nicht die gesamte Anlage außer Betrieb genommen wird, sondern nur einzelne Anlagenteile außer Betrieb genommen werden, wobei zur Außerbetriebnahme dieser Anlagenteile die folgenden Schritte durchlaufen werden:
(i) a) Stoppen der Zufuhr von Formaldehyd in den Reaktor IA);
(ii) a) Stoppen der Zufuhr von Anilin in den Reaktor IA);
(iii) a) Stoppen der Zufuhr von Säure in den Reaktor IIA);
   oder
(i) b) Stoppen der Zufuhr von Formaldehyd in den Reaktor IIB);
(ii) b) Stoppen der Zufuhr von Säure in den Reaktor IB);
(iii) b) Stoppen der Zufuhr von Anilin in den Reaktor IB);
   und
(iv) Betreiben, wobei gegebenenfalls der Waschbehälter V) und der Trennbehälter VI) ausgenommen werden, aller Anlagenteile, die nicht vom Produktionsstillstand betroffen sind, in Kreislauffahrweise durch Verwenden des Ausgangstroms eines Anlagenteils als Eingangsstrom dieses Anlagenteils oder eines anderen Anlagenteils, welcher dem betreffenden Anlagenteil vorgeschaltet ist;
(v) Außerbetriebnahme mindestens eines Anlagenteils;
(vi) Optional Öffnen des in Schritt (v) außer Betrieb genommenen mindestens einen Anlagenteils;
(vii) Durchführen einer Instandhaltungs-, Reinigungs- und/oder Reparaturmaßnahme in dem mindestens einen in Schritt (v) außer Betrieb genommenen Anlagenteil;
(viii) Optional Schließen und optional Inertisieren des mindestens einen in Schritt (v) außer Betrieb genommenen Anlagenteils.

Die Herstellung von Di- und Polyaminen der Diphenylmethanreihe (MDA) umfasst die Schritte: In einer Variante A)
IA) Reaktion von Anilin und Formaldehyd in einem Reaktor in Abwesenheit eines sauren Katalysators unter Bildung eines Aminals, wobei Anilin mit einem Massenstrom m₁ und Formaldehyd mit einem Massenstrom m₂ in den Reaktor eingetragen werden, gefolgt von Trennung des erhaltenen Reaktionsgemisches in eine wässrige und eine organische, das Aminal enthaltende Phase in einer in den Reaktor integrierten Phasentrenneinrichtung oder in einem separaten Phasentrennapparat (dem sog. "Aminalabscheider");
IIA) Reaktion zumindest eines Teils der in Schritt IA) erhaltenen organischen, das Aminal enthaltenden Phase in einem Reaktor mit Säure, wobei das Aminal zu Di- und Polyaminen der Diphenylmethanreihe reagiert;
   ("Aminal verfahren");
   oder in einer Variante B)
IB) Reaktion von Anilin und Säure in einem Reaktor;
IIB) Reaktion zumindest eines Teils des in Schritt IB) erhaltenen Reaktionsgemisches in einem Reaktor mit Formaldehyd zu Di- und Polyaminen der Diphenylmethanreihe, wobei das Anilin enthaltende Reaktionsgemisch aus Schritt IB) mit einem Massenstrom m₁ und Formaldehyd mit einem Massenstrom m₂ in den Reaktor von Schritt IIB) eingetragen werden;
   ("Neutralisationsverfahren")
   und (für beide Varianten A) und B)) die Schritte III) bis VII)
III) Neutralisation des in Schritt IIA) oder IIB) erhaltenen Reaktionsgemisches in einem Reaktor;
IV) Auftrennen des in Schritt III) erhaltenen neutralisierten Reaktionsgemisches in eine organische Phase, umfassend Di- und Polyamine der Diphenylmethanreihe und eine wässrige Phase in einem Trennbehälter;
V) Waschen der organischen Phase mit Waschflüssigkeit in einem Waschbehälter;
VI) Auftrennen des in Schritt V) erhaltenen Gemisches in eine organische Phase, umfassend Di- und Polyamine der Diphenylmethanreihe und eine wässrige Phase in einem Trennbehälter;
VII) Destillieren der organischen Phase aus Schritt VI), dabei Abtrennen der Di- und Polyamine der Diphenylmethanreihe von Wasser und Anilin, wobei ein Wasser und Anilin enthaltender Strom erhalten wird;
   sowie bevorzugt
VIII) Aufarbeitung der wässrigen Phase aus Schritt IA) und/oder der wässrigen Phase aus Schritt IV) und/oder der wässrigen Phase aus Schritt VI) und/oder des Wasser und Anilin enthaltenden Stroms aus Schritt VII) in einer Abwasseraufarbeitungseinrichtung bevorzugt umfassend einen Abwassersammelbehälter, Abwassererhitzer und Anilintrennbehälter, wobei im Fall mehrerer aufzuarbeitender wässriger Phasen bzw. Ströme diese bevorzugt im Abwassersammelbehälter vereinigt und gemeinsam der weiteren Aufarbeitung zugeführt werden.

Im Folgenden werden die Schritte IA), IB), IIA) und IIB) auch verkürzt unter den Oberbegriffen I) und II) zusammengefasst behandelt, sofern dies möglich ist.

Im Sinne der vorliegenden Erfindung werden unter *"Di- und Polyaminen der Diphenylmethanreihe"* Amine und Gemische von Aminen folgenden Typs verstanden:

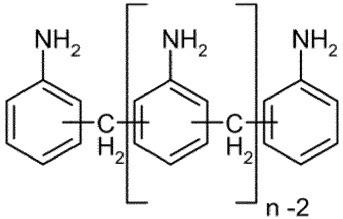

Dabei steht n für eine natürliche Zahl ≥ 2. Im Folgenden werden die Verbindungen dieses Typs, bei denen n = 2 ist, auch als Diamine der Diphenylmethanreihe oder Diaminodiphenylmethane (nachfolgend MMDA) bezeichnet. Verbindungen dieses Typs, bei denen n > 2 ist, werden im Rahmen dieser Erfindung auch als Polyamine der Diphenylmethanreihe oder Polyphenylenpolymethylenpolyamine (nachfolgend PMDA) bezeichnet. Gemische aus beiden Typen werden auch als Di- und Polyamine der Diphenylmethanreihe bezeichnet (nachfolgend MDA). Industriell werden die Di- und Polyamingemische überwiegend durch Phosgenierung zu den entsprechenden Di- und Polyisocyanaten der Diphenylmethanreihe umgesetzt.

Die *"Außerbetriebnahme"* eines Anlagenteils meint dabei dessen Stilllegung, so dass in dem Anlagenteil eine Revisions-, Reparatur-, Wartungs- oder Reinigungsmaßnahme durchgeführt werden kann. Die vorliegende Erfindung ermöglicht es, dass bei einer derartigen Maßnahme nicht die gesamte Produktionsanlage außer Betrieb genommen werden muss. Vielmehr ermöglicht es die vorliegende Erfindung, dass nicht von der Revisions-, Reparatur-, Wartungs- oder Reinigungsmaßnahme betroffene Anlagenteile bzw. die entsprechenden Verfahrensschritte in sog. "Kreislauffahrweise" betrieben werden können. Der Begriff *"Außerbetriebnahme"* umfasst demnach erfindungsgemäß bei Vorhandensein von *m Anlagenteilen im Sinne der vorliegenden Erfindung* (siehe hierzu auch den folgenden Absatz), wobei *m* eine natürliche Zahl ist, die Außerbetriebnahme von maximal *m* - 1 dieser Anlagenteile. Erfindungsgemäß wird also mindestens ein Anlagenteil **nicht** *"außer Betrieb genommen"* (d. h. *"vollständig stillgelegt"*). Bevorzugt befasst sich die vorliegende Erfindung mit dem Fall der Außerbetriebnahme von 1 bis 2 Anlagenteilen, besonders bevorzugt von 1 Anlagenteil. Erfindungsgemäß wird daher bei Außerbetriebnahme eines Anlagenteils (oder mehrerer Anlagenteile, jedoch nicht aller Anlagenteile) in jedem Fall die Bildung weiteren Produkts unterbrochen (da der Massenstrom m₂ auf null reduziert wird und daher kein weiteres Produkt mehr produziert werden kann). Erfindungsgemäß umfasst ist aber auch der Fall, dass der Reaktor aus Schritt (I) in *Kreislauffahrweise* (siehe hierzu auch den folgenden Absatz) betrieben und *ein anderes Anlagenteil* im Sinne der zuvor genannten Definition *außer Betrieb genommen* wird.

Unter *"Kreislauffahrweise"* wird im Rahmen dieser Erfindung verstanden, dass der Ausgangstrom eines Anlagenteils als Eingangsstrom dieses Anlagenteils oder eines anderen Anlagenteils, welcher dem betreffenden Anlagenteil vorgeschaltet ist (d. h. strömungstechnisch davorliegt), verwendet wird. Dabei ist mit *"Anlagenteil"* das dem jeweiligen Schritt (I) bis (VII), insofern diese durchgeführt werden, entsprechende Anlagenteil einer Anlage zur Herstellung von Di- und Polyaminen der Diphenylmethanreihe (MDA) nach dem erfindungsgemäßen Verfahren gemeint. So umfasst beispielsweise das Anlagenteil des Schritts (I) *"einen Reaktor"*, wobei dieser Begriff auch solche Ausführungsformen umfasst, in denen mehrere Reaktoren (z. B. eine Kaskade mehrere in Serie geschalteter Reaktoren) eingesetzt werden (mit anderen Worten, das Wort *"ein"* ist diesem Zusammenhang und im Zusammenhang mit anderen Apparaten als unbestimmter Artikel und nicht als Zahlwort aufzufassen). Parallel oder in Reihe geschaltete Reaktoren sind im Stand der Technik auch in der Herstellung von MDA bekannt und können in bestimmten Dimensionierungen und betrieblichen Eigenarten auch Vorteile mit sich bringen. In der Herstellung von MDA können daher in Reihe oder parallel geschaltete Reaktoren, insbesondere zu den Schritten (IA), (IIA), (IB), (IIB) bevorzugt angewendet werden.

Die Kreislauffahrweise kann auch über mehrere Apparate eines Anlagenteils eingestellt werden. Beispielsweise kann der Ausgangsstrom des letzten Apparats mehrerer in Serie geschalteter Apparate eines Anlagenteils als Eingangsstrom des ersten Apparats der in Serie geschalteten Apparate dieses Anlagenteils verwendet werden. Es ist auch möglich, die Kreislauffahrweise nur auf einen Teil der Apparate eines Anlagenteils anzuwenden, z. B. wenn der Ausgangsstrom des letzten Apparats mehrerer in Serie geschalteter Apparate eines Anlagenteils nicht in den ersten, sondern in einen weiteren Apparat dieses Anlagenteils zurückgeführt wird.

Die Kreislauffahrweise kann auch über mehrere Anlagenteile eingestellt werden. Beispielsweise kann der Ausgangsstrom des letzten Apparats eines Anlagenteils, z. B. des Trennbehälters aus Schritt IV), als Eingangsstrom des ersten Apparats eines davor liegenden Anlagenteils, z. B. des Neutralisationsreaktors aus Schritt III), verwendet werden, wobei die Kreislauffahrweise eingestellt wird, indem dann der Ausgangsstrom des beispielhaft genannten Neutralisationstrennbehälters als Eingangsstrom des Neutralisationsreaktors dient.

Nachfolgend beschriebene Ausführungsformen der Erfindung können beliebig miteinander kombiniert werden, sofern sich aus dem Kontext nicht eindeutig das Gegenteil ergibt.

Die Schritte I) und II) der MDA-Herstellung werden im Rahmen eines kontinuierlichen oder semikontinuierlichen, vorzugsweise im Rahmen eines kontinuierlichen, Prozesses durchgeführt. Durch das Abstellen von m₂, also des Massenstromes von Formaldehyd in den Reaktor aus Schritt IA) bzw. IIB), wird sichergestellt, dass während der Unterbrechung (der Außerbetriebnahme eines oder mehrerer Anlagenteile), die, wie oben beschrieben, zum Zwecke der Revision, Reparatur, Wartung und/oder Reinigung eines Teiles der Herstellungsanlage durchgeführt wird, die Reaktionen in den Schritten IA) und IIA) bzw. im Schritt IIB) nicht weiter stattfinden. Hierbei ist es in Variante A) und in Variante B) insbesondere bevorzugt, dass bei der Abstellung der Zufuhr von Formaldehyd die Zufuhr von Anilin nicht gleichzeitig mit unterbrochen wird. Vielmehr ist es bevorzugt, die Zufuhr weiteren Anilins zeitverzögert (vorzugsweise mindestens 10 min, weiter bevorzugt mindestens 20 min und besonders bevorzugt mindestens 30 min nachdem m₂ gleich null ist) zu unterbrechen und dann den Reaktor des Schritts IA) bzw. des Schritts IB) in Kreislauffahrweise zu bringen, d. h. die ausgetragene, Anilin bzw. das Reaktionsprodukt aus Anilin und Säure enthaltende Reaktionsmischung wieder als Eingangsstrom des jeweiligen Reaktors zu verwenden. Damit kann vorteilhafterweise erreicht werden, dass zum einen gegebenenfalls die Bildung von Nebenprodukten verhindert werden kann und zum anderen beispielsweise ein Verklumpen des Reaktionsgemisches unterbleibt. Damit kann effizient ein Verunreinigen des gewünschten Produktes, ein Verstopfen von Anlagenteilen wie beispielsweise Rohrleitungen, Ventilen und Pumpen und das Erzeugen von Ausschuss vermieden werden.

Zweckmäßigerweise sind in Variante A) der Reaktor in Schritt IA) und der Reaktor in Schritt IIA) voneinander verschieden ("Aminalreaktor" (Schritt IA) und "Umlagerungsreaktor" (Schritt IB)). Es ist aber nicht ausgeschlossen und von der vorliegenden Erfindung mit umfasst, dass der Reaktor in Schritt IA) und der Reaktor in Schritt IB) derselbe Reaktor sind.

In der alternativen Fahrweise gemäß Variante B) wird zweckmäßigerweise die Reaktion von Anilin und Säure, beispielsweise Salzsäure (Schritt IB)) in einem ersten Reaktor und die Reaktion des Reaktionsgemisches aus Schritt IB) mit Formaldehyd in einem zweiten Reaktor durchgeführt (Schritt IIB)). Es ist aber nicht ausgeschlossen und von der vorliegenden Erfindung mit umfasst, dass die Schritte IB) und IIB) im selben Reaktor durchgeführt werden.

Durch das erfindungsgemäße Verfahren zum Betrieb einer Anlage zur Herstellung von MDA ergeben sich die folgenden Vorteile:
i) Erhöhung der Produktivität, weil sich die Verfügbarkeit der Anlage erhöht, da sich der Zeitbedarf für das Abfahren und Wiederanfahren der Anlage für den Produktionsstillstand stark minimiert.
ii) Investkosten in eine größere Anlagenkapazität entfallen.
iii) Investkosten in einen größeren Endprodukttank zur Abpufferung von längeren Stillstandszeiten entfallen.
iv) Vermeidung von überflüssigen Abfallprodukten (überschüssiges Anilin, Roh-MDA, Abwasser, die zusätzlich aufgereinigt werden müssen), die entstehen, wenn die Anlage komplett neu angefahren werden muss.
v) In vielen Fällen kommt es zur Einsparung von Energie, weil die zum Wiederanfahren benötigten Vorbereitungen für die ausgeschalteten Anlagenteile wie das Aufwärmen der Einsatz- und Hilfsstoffe oder das Aufwärmen des Equipments etc. entfallen.
vi) In vielen Fällen kommt es zur Einsparung von Hilfsstoffen wie Kondensat und Stickstoff.
vii) Die Reparaturanfälligkeit von Pumpen oder Kompressoren wird vermindert, da, wenn diese bei einem Stillstand abgestellt werden, bei jedem Wiederanfahren deren Gleitringdichtungen leiden. Somit werden Folgereparaturen vermieden, was sich wiederum positiv auf die Produktivität der Anlage und die Instandhaltungskosten auswirkt.

Sollten dabei zwei oder mehr MDA-Produktionsstraßen bzw. Reaktorlinien parallel betrieben werden, dann können zunächst in einer Produktionsstraße bzw. Reaktorlinie erfindungsgemäß ein oder mehrere Anlagenteile außer Betrieb genommen werden und die andere(n) Produktionsstraße(n) bzw. Reaktorlinie(n), insofern erforderlich, nacheinander im Hinblick auf die Außerbetriebnahme zugehöriger Anlagenteile, die nicht vom Produktionsstillstand betroffen sind, erfindungsgemäß betrieben werden. Alternativ ist es im Rahmen der vorliegenden Erfindung aber auch möglich, alle MDA-Produktionsstraßen bzw. Reaktorlinien, insofern erforderlich, gleichzeitig oder zeitnah gemäß dem erfindungsgemäßen Verfahren im Hinblick auf die Außerbetriebnahme eines oder mehrerer zugehöriger Anlagenteile zu betreiben.

Die Herstellung von Di- und Polyaminen der Diphenylmethanreihe **im Normalbetrieb** lässt sich exemplarisch wie folgt zusammenfassen:
In Variante A)
   a) Kernvorgang des Schritts IA): Anilin und Formaldehyd werden in Abwesenheit eines sauren Katalysators zu Aminal und Wasser kondensiert, und das dabei entstehende Aminal wird aus dem Aminalreaktor ausgetrieben,
   b) Wasser aus Schritt a), das im Wesentlichen aus Kondensationswasser der Aminalreaktion und Wasser aus dem Einsatzstoff Formaldehyd herrührt, wird zumindest teilweise aus der Aminalreaktionslösung als wässrige Phase abgetrennt,
   c) Kernvorgang des Schritts IIA): das Aminal aus Schritt b) wird säurekatalysiert zu MDA umgelagert,
In Variante B)
   a') Kernvorgang des Schritts IB): Anilin und Salzsäure werden zu einem Gemisch aus Anilin und Anilinhydrochlorid umgesetzt,
   b') Kernvorgang des Schritts IIB): das Gemisch aus Anilin und Anilinhydrochlorid aus Schritt a') wird mit Formaldehyd zu MDA umgesetzt,
In beiden Varianten A) und B)
   d) Kernvorgang des Schritts III): das Reaktionsgemisch enthaltend Di- und Polyamine der Diphenylmethanreihe aus Schritt IIA) oder Schritt IIB) wird neutralisiert,
   e) Kernvorgang des Schritts IV): das neutralisierte Reaktionsgemisch enthaltend Di- und Polyamine der Diphenylmethanreihe wird in einem Trennbehälter in eine organische Phase enthaltend Di- und Polyamine der Diphenylmethanreihe und eine wässrige Phase aufgetrennt,
   f) Kernvorgang des Schritts V): die organische Phase enthaltend Di- und Polyamine der Diphenylmethanreihe wird in einem Waschbehälter mit Waschflüssigkeit weiter gereinigt,
   g) Kernvorgang des Schritts VI): das so erhaltene Gemisch wird in einem Trennbehälter in eine organische Phase enthaltend Di- und Polyamine der Diphenylmethanreihe und eine wässrige Phase aufgetrennt,
   h) Kernvorgang des Schritts VII): die gewaschene Di- und Polyamine der Diphenylmethanreihe enthaltende organische Phase wird destillativ von Wasser und Anilin befreit.

Die Kondensation von Anilin und Formaldehyd in a) kann nach einem Verfahren nach dem Stand der Technik durchgeführt werden. Dabei werden normalerweise Anilin und wässrige Formaldehydlösung bei Molverhältnissen im Bereich von 1,5 bis 20, bevorzugt 1,5 bis 10 und besonders bevorzugt 1,5 bis 6 bei Temperaturen von 20 °C bis 120 °C, bevorzugt 40 °C bis 110 °C und besonders bevorzugt 60 °C bis 100 °C zu Aminal und Wasser kondensiert. Die Umsetzung erfolgt üblicherweise bei Umgebungsdruck. Sie kann aber auch bei leichtem Überdruck durchgeführt werden. Geeignete Anilinqualitäten sind z. B. beschrieben in EP 1 257 522 B1, EP 2 103 595 A1 und EP 1 813 598 B1. Es werden bevorzugt technische Qualitäten an Formalin (wässrige Lösung von Formaldehyd) mit 30 Massen-% bis 50 Massen-% Formaldehyd in Wasser eingesetzt. Jedoch sind auch Formaldehydlösungen mit niedrigeren oder höheren Konzentrationen oder auch der Einsatz gasförmigen Formaldehyds denkbar.

In b) kann die Phasentrennung von organischer Aminalphase und wässriger Phase bei Temperaturen von 20 °C bis 120 °C, bevorzugt von 40 °C bis 110 °C, besonders bevorzugt von 60 °C bis 100 °C, bevorzugt bei Umgebungsdruck erfolgen. Die Phasentrennung kann aber auch bei leichtem Überdruck durchgeführt werden.

Die Umlagerung des Aminals in c) kann in Gegenwart eines sauren Katalysators erfolgen, üblicherweise einer starken Mineralsäure wie Salzsäure. Bevorzugt ist der Einsatz von Mineralsäure in einem molaren Verhältnis Mineralsäure zu Anilin von 0,001:1 bis 0,9:1, bevorzugt 0,05:1 bis 0,5:1. Es können natürlich auch feste, saure Katalysatoren, wie in der Literatur beschrieben, verwendet werden. Dabei kann Formaldehyd in eine Mischung von Anilin und saurem Katalysator gegeben und die Reaktionslösung durch stufenweises Erhitzen abreagiert werden. Alternativ können auch Anilin und Formaldehyd zunächst vorreagiert werden und anschließend mit oder ohne vorhergehender Wasserabtrennung mit dem sauren Katalysator oder einem Gemisch von weiterem Anilin und saurem Katalysator versetzt werden, wonach die Reaktionslösung durch stufenweises Erhitzen abreagiert wird. Diese Reaktion kann kontinuierlich, semikontinuierlich oder diskontinuierlich nach einem der zahlreichen in der Literatur beschriebenen Verfahren ausgeführt werden (z. B. in EP 1 616 890 A1 oder EP 127 0544 A1). In d) kann das Reaktionsgemisch enthaltend die Di- und Polyamine der Diphenylmethanreihe ggf. unter Zusatz von Wasser und/oder Anilin neutralisiert werden. Nach dem Stand der Technik erfolgt die Neutralisation üblicherweise bei Temperaturen von beispielsweise 90 °C bis 100 °C ohne Zusatz weiterer Substanzen. Sie kann aber auch auf einem anderen Temperaturniveau erfolgen, um z. B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung. Die zur Neutralisation eingesetzte Base wird bevorzugt in Mengen von größer als 100 %, besonders bevorzugt 105 % bis 120 % der für die Neutralisation des eingesetzten sauren Katalysators stöchiometrisch benötigten Menge eingesetzt (siehe EP 1 652 835 A1).

Anschließend kann in e) das neutralisierte Reaktionsgemisch enthaltend die Di- und Polyamine der Diphenylmethanreihe in eine organische Phase enthaltend Di- und Polyamine der Diphenylmethanreihe und eine wässrige Phase aufgetrennt werden. Dies kann durch die Zugabe von Anilin und/oder Wasser unterstützt werden. Wird die Phasentrennung durch Zugabe von Anilin und/oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und durch Zugabe von Anilin und/oder Wasser verdünnte Reaktionsgemisch wird anschließend bevorzugt einem Apparat zugeführt, der aufgrund seiner Konfiguration und/oder Einbauten besonders zur Auftrennung in eine organische Phase enthaltend MDA und eine wässrige Phase geeignet ist, bevorzugt Phasentrenn- oder Extraktionsvorrichtungen entsprechend dem Stand der Technik, wie sie beispielsweise in Mass-Transfer Operations, 3rd Edition, 1980, McGraw-Hill Book Co, S. 477 bis 541, oder Ullmann's Encyclopedia of Industrial Chemistry (Vol. 21, Liquid-Liquid Extraction, E. Müller et al., Seite 272-274, 2012 Wiley-VCH Verlag GmbH & Co. KGaA, Weinheim, DOI: 10.1002/14356007.b03_06.pub2) oder in Kirk-Othmer Encyclopedia of Chemical Technology (siehe "http://onlinelibrary.wiley.com/book/10.1002/0471238961", Published Online: 15 Juni 2007, Seite 22-23) beschrieben sind (Mixer-Settler-Kaskade oder Absetzbehälter).

In f) kann sich eine Wäsche der organischen Phase mit Wasser und in g) eine neuerliche Abscheidung der Wasserphase zur Entfernung von Restgehalten an Salz (bevorzugt wie in DE-A-2549890, Seite 3 beschrieben) anschließen. Die in Schritt g) erhaltene organische Phase hat bevorzugt eine Zusammensetzung, bezogen auf das Gewicht des Gemisches, von 5 bis 15 Gewichtsprozent Wasser, und, je nach Einsatzverhältnissen von Anilin und Formaldehyd, 5 bis 90 Gewichtsprozent, bevorzugt 5 bis 40 Gewichtsprozent Anilin und 5 bis 90 Gewichtsprozent, bevorzugt 50 bis 90 Gewichtsprozent Di- und Polyamine der Diphenylmethanreihe. Nach Austritt aus der Phasentrennung in Schritt g) hat die organische Phase enthaltend Di- und Polyamine der Diphenylmethanreihe üblicherweise eine Temperatur von 80 °C bis 150 °C.

In h) kann aus der in g) erhaltenen organischen Phase enthaltend Di- und Polyamine der Diphenylmethanreihe destillativ Wasser und Anilin abgetrennt werden, wie in EP 1 813 597 B1 beschrieben.

Die so erhaltenen Di- und Polyamine der Diphenylmethanreihe können nach den bekannten Methoden unter inerten Bedingungen mit Phosgen in einem organischen Lösungsmittel zu den entsprechenden Di- und Polyisocyanaten der Diphenylmethanreihe, dem MDI, umgesetzt werden. Dabei kann die Phosgenierung nach einem der aus dem Stand der Technik bekannten Verfahren durchgeführt werden (z. B. DE-A-844896 oder DE-A-19817691).

In einer bevorzugten Ausführungsform beträgt in Schritt IA) oder IB) der Massenstrom m₁ ≥ 1000 kg/Stunde.

In einer weiteren bevorzugten Ausführungsform beträgt in Schritt (IA) oder (IIB) der Massenstrom m₂ ≥ 300 kg/Stunde.

In beiden Varianten kann der eingesetzte Formaldehyd aus jedem aus dem Stand der Technik bekannten Verfahren zur Herstellung von Formaldehyd stammen. Lediglich exemplarisch sei an dieser Stelle das Silberkontakt-Verfahren erwähnt.

In einer weiteren Ausführungsform des erfindungsgemäßen Verfahrens liegt der Reaktor des Schritts IA) bzw. des Schritts IIB) zumindest teilweise mit Anilin bzw. dem Reaktionsprodukt aus Anilin Säure befüllt vor. Beim Befüllen der Anlagenteile mit Anilin bzw. dem Reaktionsprodukt aus Anilin und Säure kann in Abwesenheit von Formaldehyd eine Unterbrechung der Produktion ohne die Bildung unerwünschter hochmolekularer Nebenprodukte erfolgen.

Eine typische **Anlage zur Herstellung von MDA** kann gemäß dem Stand der Technik üblicherweise in folgende Anlagenteile aufgeteilt werden: Einen Reaktor zur Aminalreaktion (IA)) bzw. zur Umsetzung von Anilin Säure (IB)), einen Reaktor zur Umlagerungsreaktion (IIA)) bzw. zur Reaktion des Produkts aus IB) mit Formaldehyd, des Weiteren Anlagenteile für die Neutralisation, Wäsche, Destillation und Abwasseraufarbeitung.

Eine Anlage zur Herstellung von Di- und Polyaminen der Diphenylmethanreihe (MDA), die mit dem erfindungsgemäßen Verfahren betrieben werden kann, umfasst die Anlagenteile
IA) einen Reaktor mit integrierter Phasentrenneinrichtung oder einen Reaktor und einen separaten Phasentrennapparat zur Reaktion von Anilin und Formaldehyd in Abwesenheit eines sauren Katalysators unter Bildung eines Aminals und anschließender Trennung des erhaltenen Reaktionsgemisches in eine wässrige und eine organische, das Aminal enthaltenden Phase und
IIA) einen Reaktor zur Reaktion der im Reaktor IA) erhaltenen organischen, das Aminal enthaltenden Phase mit Säure;
   oder
IB) einen Reaktor zur Reaktion von Anilin mit Säure und
IIB) einen Reaktor zur Reaktion des im Reaktor IB) erhaltenen Reaktionsgemisches mit Formaldehyd;
III) einen Reaktor zur Neutralisation des Reaktionsgemisches aus IIA) oder IIB);
IV) einen Trennbehälter zum Auftrennen des neutralisierten Reaktionsgemisches aus III) in eine organische Phase, umfassend Di- und Polyamine der Diphenylmethanreihe und eine wässrige Phase;
V) einen Waschbehälter zum Waschen der organischen Phase aus IV) mit Waschflüssigkeit;
VI) einen Trennbehälter zum Auftrennen des Gemisches aus V) in eine organische Phase, umfassend Di- und Polyamine der Diphenylmethanreihe und eine wässrige Phase;
   und
VII) eine Destillationseinrichtung zum Destillieren der organischen Phase aus VI) unter Erhalt von Di- und/oder Polyaminen der Diphenylmethanreihe und eines Wasser und Anilin enthaltenden Stroms;
   sowie optional
VIII) eine Abwasseraufarbeitungseinrichtung zur Aufarbeitung der wässrigen Phase aus IA) und/oder der wässrigen Phase aus IV) und/oder der wässrigen Phase aus VI) und/oder des Wasser und Anilin enthaltenden Stroms aus VII), bevorzugt umfassend einen Abwassersammelbehälter, Abwassererhitzer und Anilintrennbehälter.

Bevorzugt umfasst die Anlage auch Anlageteil VIII).

Die Anlagenteile können bevorzugt unabhängig voneinander auf aus rückgeführten Ausgangsströmen bestehenden Eingangsströmen geschaltet werden. Die Anlagenteile können auch gleichzeitig auf aus rückgeführten Ausgangsströmen bestehenden Eingangsströmen geschaltet werden. Dabei ist es vorgesehen, dass in jedem anderen, nicht betroffenen Anlagenteil der Ausgangsstrom zurückgeführt und als Eingangsstrom des jeweiligen Anlagenteils verwendet wird, wobei die Anlagenteile zur Wäsche des Produktstromes (Waschbehälter V) und Trennbehälter VI) hiervon ausgenommen sein können, also bevorzugt bei einer Unterbrechung des Verfahrens lediglich abgestellt, aber nicht auf Kreislauffahrweise eingestellt werden.

Vorzugsweise umfasst der **Reaktor IA**), der Aminalreaktor, eine Anilindosierleitung zum Reaktor, ein Siphon und einen Aminalabscheider. Der Reaktor IA) kann ferner einen Aminalkühler umfassen. Vorzugsweise umfasst der **Reaktor IIB**) eine absperrbare Dosierleitung für das Reaktionsprodukt aus Anilin und Säure (oder, falls Reaktor IIB und IB identisch sind, je eine getrennte Dosierleitung für Anilin und Säure), eine absperrbare Dosierleitung für Formaldehyd, ein Mischaggregat (bevorzugt einen Rührer) und einen Wärmeaustauscher zur Regelung der Temperatur der Reaktionsmischung. Es ist bevorzugt, die Anlage so zu betreiben, dass bei Außerbetriebnahme eines oder mehrerer vom Reaktor IA) bzw. vom Reaktor IIB) verschiedener Anlagenteile zuerst der Formaldehydstrom in den Reaktor IA) bzw. in den Reaktor IIB) und danach, vorzugsweise mindestens 10 min, weiter bevorzugt mindestens 20 min und besonders bevorzugt mindestens 30 min danach, der Anilinstrom bzw. der Strom enthaltend das Reaktionsprodukt aus Anilin und Säure (oder, falls Reaktor IIB und IB identisch sind, der Anilinstrom und anschließend der Säurestrom), abgestellt wird. In Variante A) wird dann das mit Anilin verdünnte Aminal ungekühlt vom Aminalreaktor über den Siphon in den Aminalabscheider und von dort aus im Kreis zurück über die Anilindosierleitung zum Aminalreaktor für unbestimmte bzw. zumindest für die gewählte Zeit umgepumpt. In der Variante B) wird dann das Mischaggregat abgestellt.

Vorzugsweise umfasst der **Reaktor IIA**), in dem in Variante A) die säurekatalysierte Umlagerungsreaktion stattfindet, eine Reaktorkaskade von mehreren Umlagerungsreaktoren und Verweilzeittürmen, also beispielsweise einen ersten Umlagerungstank, eine Aminalleitung und eine Umlagerungskaskade. Vorzugsweise umfasst der **Reaktor IB**), in dem in Variante B) die Reaktion von Anilin und Säure stattfindet, eine absperrbare Anilindosierleitung, eine absperrbare Säuredosierleitung, ein Mischaggregat, besonders bevorzugt einen Rührer, und einen Wärmeaustauscher zur Regelung der Temperatur der Reaktionsmischung. Es ist bevorzugt, die Anlage so zu betreiben, dass bei einer Außerbetriebnahme eines oder mehrerer vom Reaktor IIA) bzw. vom Reaktor IB) verschiedener Anlagenteile zuerst der Säurestrom, vorzugsweise ein Salzsäure-Strom, in den Reaktor IIA) bzw. in den Reaktor IB) und anschließend der Aminalstrom bzw. der Strom umfassend Anilin in den Reaktor IIA) bzw. in den Reaktor IIB) abgestellt werden. In Variante A) wird dann die den Ausgangsstrom des Reaktors IIA) bildende Kondensationslösung, umfassend Roh-MDA, Anilin und deren Hydrochloride, vorzugsweise ohne Beheizung vom letzten Umlagerungsreaktor in den ersten Umlagerungsreaktor, oder gegebenenfalls in den zweiten, dritten etc. Umlagerungsreaktor und über die (restliche) Reaktorkaskade und die Aminalleitung für unbestimmte Zeit bzw. zumindest für die gewählte Zeit im Kreis geführt. In Variante B) wird dann vorzugsweise das Mischaggregat (bevorzugt einen Rührer) abgestellt. Das Führen der Kondensationslösung in Variante A wird bevorzugt mittels Pumpen durchgeführt.

Des Weiteren ist es bevorzugt, dass der **Reaktor zur Neutralisation III**) in der Anlage eine Basendosiereinrichtung (bevorzugt eine Natronlaugedosiereinrichtung), einen Neutralisationsrührbehälter und einen Neutralisationskondensator zum Kondensieren der entstehenden Brüden umfasst. Ferner ist es bevorzugt, dass der **Trennbehälter IV**) einen Neutralisationsabscheider mit Kreislaufpumpe für die untere alkalische, wässrige Phase umfasst. Die Anlage wird bevorzugt so betrieben, dass bei Außerbetriebnahme eines oder mehrerer vom Reaktor III) und Trennbehälter IV) verschiedener Anlagenteile zuerst die saure Kondensationslösung und danach, vorzugsweise 5 min später, weiter bevorzugt 10 min später und besonders bevorzugt 15 min später, der Zufluss der Base (bevorzugt Natronlauge oder Ammoniak, besonders bevorzugt Natronlauge) und der Zufluss des Waschwassers abgestellt werden. Somit kann das im Neutralisationsabscheider befindliche Gemisch für unbestimmte Zeit mittels der Kreislaufpumpe aus dem Neutralisationsabscheider in den Neutralisationsrührbehälter über ein Siphon zurück in den Neutralisationsabscheider gepumpt werden und somit auf diese Weise dieser Teil der Anlage bzw. des Verfahrensschrittes im Kreis gefahren werden.

Bevorzugt umfasst der **Waschbehälter V**) der Anlage einen Roh-MDA-Wäscher und eine Waschwasserzugabe. Der **Trennbehälter VI**) der Anlage umfasst einen Phasentrennapparat zum Auftrennen des im Waschbehälter V) gebildeten Gemisches in eine organische, MDA enthaltende Phase und in eine wässrige Phase. Die Anlage wird bevorzugt so betrieben, dass bei einer Außerbetriebnahme eines oder mehrerer vom Waschbehälter V) und Trennbehälter VI) verschiedener Anlagenteile die Waschwasserzugabe, bestehend aus Kondensat und/oder dem Seitenstrom der Prozessabwasserkolonne, abgestellt und das im Waschbehälter V) und Trennbehälter VI) jeweils befindliche Gemisch stehengelassen wird. Eine Kreislauffahrweise unterbleibt in diesen Anlagenteilen vorzugsweise.

Bevorzugt umfasst die **Destillationseinrichtung VII**) der Anlage eine Destillationsanlage mit Vakuumsystem, umfassend eine Pumpenvorlage der Destillation für das Anilin- und wasserhaltige Roh-MDA, einen Wärmeaustauscher, eine Vordestillationskolonne mit Kondensationssystem zum Entfernen von überschüssigem Anilin, Wasser und Leichtsiedern, eine MDA-Kolonne mit Sumpfentnahme des Endproduktes MDA und einen Dampferzeuger. Es ist bevorzugt, die Anlage so zu betreiben, dass bei Außerbetriebnahme eines oder mehrerer von der Destillationseinrichtung VII) verschiedener Anlagenteile nach Abstellung der Wäsche kein Roh-MDA in die Pumpenvorlage der Destillation mehr eingetragen wird. Es ist daher bevorzugt, den Sumpfablauf der MDA-Kolonne über den Dampferzeuger und den Wärmeaustauscher zurück auf die Pumpenvorlage der Destillation zu führen, und somit diesen Teil der Anlage über die Pumpenvorlage, den Wärmeaustauscher die Vordestillationskolonne und in den Sumpf der MDA-Kolonne im Kreis zu fahren. Danach kann der Dampf zur Vordestillationskolonne und MDA-Kolonne abgestellt werden. Anschließend kann das Vakuumsystem der beiden Kolonnen abgestellt werden. Somit kann die Kreislauffahrweise ohne Beheizung und Vakuum für eine beliebige Zeit betrieben werden.

Werden die vorhergehenden Anlagenteile erfindungsgemäß auf Kreislauffahrweise eingestellt, fällt in der bevorzugt vorhandenen **Abwasseraufarbeitung VIII**) kein Abwasser mehr an. Bevorzugt umfasst die Abwasseraufarbeitung der erfindungsgemäßen Anlage einen Abwassersammelbehälter, Abwassererhitzer und Anilintrennbehälter. Bevorzugt wird die Anlage so betrieben, dass bei Außerbetriebnahme eines oder mehrerer von der Abwasseraufarbeitung verschiedener Anlagenteile die Abwasserextraktion in Kreislauffahrweise gestellt wird, indem der Ablauf des Anilintrennbehälters auf den Abwassersammelbehälter geführt und mittels einer oder mehreren Pumpen über den Prozessabwassererhitzer in den Anilintrennbehälter im Kreis gepumpt wird. Diese Kreislauffahrweise kann ohne Beheizung über einen beliebigen Zeitraum betrieben werden.

Darüber hinaus kann die Anlage bzw. das erfindungsgemäße Verfahren zum Betrieb der Anlage noch eine **Abwasserdestillation** umfassen, welche einen Wärmeaustauscher, eine Prozessabwasserdestillationskolonne mit Kondensationssystem, einen Prozessabwasserkühler und eine Seitenstromvorlage der Prozessabwasserdestillationskolonne umfassen kann. Die Abwasserdestillation kann erfindungsgemäß bei einer Unterbrechung des MDA-Herstellungsverfahrens beispielsweise einfach durch Abstellen des Dampfes zur Kolonne abgestellt werden. Eine Kreislauffahrweise der Abwasserdestillation kann, muss aber nicht, durchgeführt werden.

Diese bevorzugten Ausführungsformen stehen natürlich nur exemplarisch für eine Vielzahl möglicher Kreislauffahrweisen, deren genaue Ausgestaltung von den konkreten Gegebenheiten einer Produktionsanlage abhängt, aber im Sinne der vorliegenden Erfindung einfach an diese konkreten Gegebenheiten angepasst werden kann. Ein gemeinsames Merkmal aller denkbaren Kreislauffahrweisen ist jedoch, dass kein Produkt die Anlage verlässt, wenn es sich um eine einstrassige MDA-Linie handelt.

Sollten zwei oder mehr MDA-Reaktorlinien parallel betrieben werden, dann kann, muss aber nicht, Produkt die Anlage verlassen, wenn z. B. die Anlage mit Teillast gefahren wird.

Mit dem erfindungsgemäßen Verfahren zum Betrieb einer Anlage zur Herstellung von MDA kann vorteilhafterweise die Anlage bei den oben beschriebenen Unterbrechungen (Außerbetriebnahme einzelner Anlagenteile) in Kreislauffahrweise betrieben und damit die erfindungsgemäßen Vorteile und Effekte erzielt werden. In Schritt (iv) werden, gegebenenfalls mit Ausnahme des Waschbehälters V) und des Trennbehälters VI), alle Anlagenteile, die sich auf Kreislauffahrweise schalten lassen und nicht in irgendeiner Art vom Produktionsstillstand betroffen sind, in Kreislauffahrweise gebracht, wobei bevorzugt der Waschbehälter V) und der Trennbehälter VI) von der Kreislauffahrweise ausgenommen werden.

In einer bevorzugten Ausführungsform umfasst das Verfahren die weiteren Schritte
(ix) Anfahren des mindestens einen in Schritt (v) außer Betrieb genommenen Anlagenteils,
(x) Starten der Zufuhr von Formaldehyd in den Reaktor IA) oder IIB) und Starten der Zufuhr von Anilin in den Reaktor IA) oder IB) und Zufuhr von Säure in den Reaktor IIA) oder IB).

Im Folgenden sollen dazu beispielhaft für die Variante A) das Einstellen der Anlage auf Kreislauffahrweise und ebenso beispielhaft das Wiederanfahren der Anlage aus der Kreislauffahrweise heraus in den Normalbetrieb beschrieben werden:
Im ersten Schritt wird die Zufuhr von Formaldehyd in den Aminalreaktor gestoppt. Für einen gewissen Zeitraum wird Anilin zur Verdünnung der Reaktionslösung im Aminalreaktor weitergefahren. Dann wird Anilin abgeschaltet und der Aminalbereich wird in Kreislauffahrweise gestellt.

Im zweiten Schritt werden die Umlagerungsreaktoren, nachdem Salzsäure und Aminal abgestellt wurden, in Kreislauffahrweise gestellt.

Im dritten Schritt werden die Neutralisation und die Wäsche im Kreis gefahren, und das noch in der Destillation befindliche MDA wird mit Anilin verdünnt und anschließend die Destillation in Kreislauffahrweise gestellt.

Das beispielsweise von einer Instandhaltungsmaßnahme betroffene Anlagenteil wird entleert, gereinigt und gegebenenfalls für die anstehende Maßnahme geöffnet. Dann wird die Instandhaltungsmaßnahme durchgeführt und der Anlagenteil wird wieder geschlossen, gegebenenfalls inertisiert und mit Hilfsstoffen und Einsatzmaterialien befüllt und zum Anfahren vorbereitet.

Das Wiederanfahren der Anlage aus der Kreislauffahrweise heraus kann also beispielsweise wie folgt ablaufen:
Um die Anlage wieder anzufahren, geht man unter umgekehrten Vorzeichen vor. Da die Behälter und Apparate sehr voll mit verdünnter Produktionslösung beladen sind, wird zuerst die Destillation, dann die Neutralisation und Wäsche, anschließend die Umlagerungsreaktion und am Ende die Aminalreaktion wieder angefahren. Die Abwasseraufarbeitung wird in Betrieb genommen, sobald die Neutralisation läuft.

Als erstes wird in der Destillation mit Vakuumsystem, umfassend eine Pumpenvorlage der Destillation für das Anilin und Wasser haltige Roh-MDA, einen Wärmeaustauscher, eine Vordestillationskolonne mit Kondensationssystem zum Entfernen von überschüssigem Anilin, Wasser und Leichtsiedern, MDA-Kolonne mit Sumpfentnahme des Endproduktes MDA und einen Dampferzeuger, zum Anfahren nach der Abstellung zuerst das Vakuumsystem der Vordestillationskolonne und MDA-Kolonne in Betrieb genommen. Dann wird der Dampf zur Vordestillationskolonne und MDA-Kolonne geöffnet und die Kolonnen werden aufgeheizt. Nun ist das Betriebssegment der Destillation bereit, Roh-MDA aufzunehmen.

Anschließend wird in der Neutralisation, umfassend Natronlaugedosiereinrichtung, Neutralisationsrührbehälter, Neutralisationskondensator zum Kondensieren der entstehenden Brüden, Neutralisationsabscheider mit Kreislaufpumpe für die untere alkalische, wässrige Phase, zuerst die Natronlauge und das Waschwasser und 10 Minuten später die saure Kondensationslösung angestellt. Nun ist das Betriebssegment der Neutralisation angestellt, und Roh-MDA geht zur Wäsche.

Anschließend wird in der Wäsche, umfassend MDA-Wäscher, Phasentrennapparat und Waschwasserzugabe, die Waschwasserzugabe, umfassend Kondensat und/oder dem Seitenstrom der Prozessabwasserkolonne, angestellt. Nun läuft das Betriebssegment der Wäsche, und Roh-MDA wird zur Destillation geführt.

Sobald die Neutralisation und Wäsche läuft, fällt in der Abwasseraufarbeitung, umfassend eine Abwasserextraktion und eine Abwasserdestillation, nach dem Anstellen der oben genannten Anlagenteile, wieder Prozesswasser an. Um in der Abwasserextraktion Spuren an MDA mittels Anilin aus dem Prozessabwasser zu extrahieren, wird die Abwasserextraktion, die aus einem Abwassersammelbehälter, Abwassererhitzer und Anilintrennbehälter besteht, angestellt. Dazu wird das anfallende Abwasser aus den vorbeschriebenen Verfahrensschritten (Neutralisation, Wäsche und Destillation), das im Abwassersammelbehälter ankommt, mittels Pumpe über den Prozessabwassererhitzer in den Anilintrennbehälter gefahren. Von dort aus geht das extrahierte Abwasser in die Abwasserdestillation. Die Abwasserdestillation, umfassend einen Wärmeaustauscher, eine Prozessabwasserdestillationskolonne mit Kondensationssystem, einen Prozessabwasserkühler und eine Seitenstromvorlage der Prozessabwasserdestillationskolonne, wird nun angestellt, indem der Dampf zur Kolonne geöffnet wird und gereinigtes Prozessabwasser die Produktionsanlage verlässt.

Anschließend wird in der Umlagerungsreaktion, umfassend eine Reaktorkaskade von mehreren Umlagerungsreaktoren und Verweilzeittürmen, der Aminalstrom angestellt und anschließend der HCl-Strom in Betrieb genommen. Die Kondensationslösung, umfassend MDA, Anilin und Salzsäure wird beheizt. Nun ist das Betriebssegment der Umlagerungsreaktion angestellt, und Roh-MDA geht zur Neutralisation.

Anschließend wird in der Aminalreaktion, umfassend Aminalreaktor, Aminalkühler und Aminalabscheider, unter Rühren als erstes der Anilinstrom und im Anschluss der Formalinstrom angestellt. Der Aminalkühler wird in Betrieb genommen und die Aminallösung aus dem Aminalreaktor wird über den Aminalkühler gefahren und geht dann gekühlt vom Aminalreaktor über den Siphon in den Aminalabscheider. Von dort geht die organische Phase, umfassend Aminallösung, in den ersten Reaktor der Umlagerungsreaktion. Das im Aminalabscheider anfallende Aminalwasser wird der Abwasseraufarbeitung zugeführt. Nun ist das Betriebssegment der Aminalreaktion angestellt, und Aminallösung geht zur Umlagerungsreaktion.

Die komplette MDA-Anlage läuft jetzt bevorzugt mit reduzierter Last (Anfahrlast) und kann nun auf die gewünschte Sollproduktion hochgefahren werden. Es ist hier besonders bevorzugt die Produktionsanlage mit reduzierter Last anzufahren, da ansonsten die benötigten Temperaturprofile für Aminal- und Umlagerungsreaktion, Neutralisation, Wäsche und Abwasseraufarbeitung und Destillation nicht schnell genug zur Verfügung stehen könnten, was zu unvollständigen Reaktionen, vermehrten Nebenprodukten und mangelhafter Aufarbeitung des Produktes führen würde.

In der Anlage und dem erfindungsgemäßen Verfahren kann dabei jedes Anlagenteil beispielsweise per Hand auf Kreislauffahrweise eingestellt werden. In einer bevorzugten Ausführungsform erfolgt das Umstellen auf Kreislauffahrweise, das Anfahren sowie die Überwachung aller Schritte über eine zentrale Steuerungsanlage, die insbesondere bevorzugt prozessleittechnische Einrichtungen umfasst.

Der Erfolg der erfindungsgemäßen Vorgehensweise ist für den Fachmann überraschend, weil er, um grundsätzlich Energie einzusparen und um sich auf die anstehenden Instandhaltungsmaßnahmen im Produktionsstillstand konzentrieren zu können, viel eher dazu tendieren würde, *die Gesamtanlage* abzustellen, zumal für das erfindungsgemäße Verfahren bzw. für die erfindungsgemäße Anlage Zusatzinvestitionen in rückführende Rohrleitungen samt Pumpen, Umbauten an den Apparaten sowie zusätzliche Prozessleittechnik in Kauf genommen werden.

Im Folgenden soll die vorliegende Erfindung durch weitere Beispiele verdeutlicht werden.

### Beispiele:

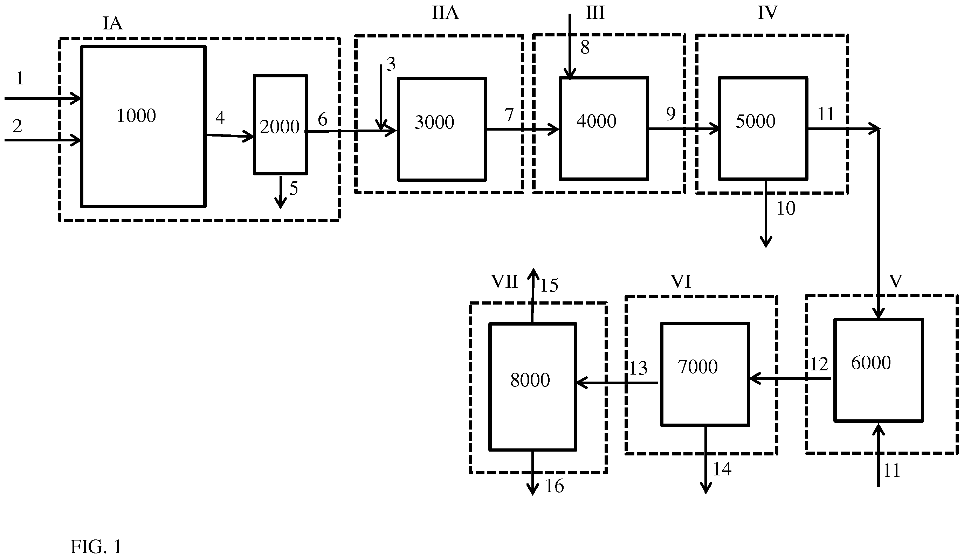

### Allgemeine Bedingungen für die Herstellung von MDA bei einer "eingefahrenen" Produktionsanlage (FIG. 1)

In einem kontinuierlichen Reaktionsprozess (Schritt a)) werden 24,3 t/h Einsatzanilin (Strom 1, enthaltend 90 Massen-% Anilin) und 9,9 t/h 32 %ige wässrige Formalinlösung (Formaldehyd, Strom 2) (molares Verhältnis Anilin zu Formaldehyd 2,1 zu 1) vermischt und bei 90 °C und 1,4 bar (absolut) in einem gerührten Reaktionskessel (1000) zum Aminal umgesetzt. Der Reaktionskessel ist mit einem Kühler mit Kühlkreislaufpumpe versehen. Das den Reaktionskessel verlassende Reaktionsgemisch (4) wird in einen Phasentrennapparat (Aminalabscheider, 2000) geführt (Schritt b)). Nach der Phasentrennung zur Entfernung der wässrigen Phase (5), die zu einem Abwassersammelbehälter (nicht gezeigt) geführt wird, wird die organische Phase (6) in einer Mischdüse mit 30 %iger wässriger Salzsäure (3) versetzt (Protonierungsgrad 10 %, d. h., pro Mol Aminogruppen werden 0,1 Mol HCl zugegeben) und in den ersten Umlagerungsreaktor gefahren. Die Umlagerungsreaktion wird in einer Reaktorkaskade (3000) bei 45 °C bis 165 °C durchgeführt (Schritt c)). Nach vollständiger Reaktion wird das erhaltene Reaktionsgemisch (7) mit 32 %iger Natronlauge (8) im molaren Verhältnis von 1,1:1 Natronlauge zu HCl versetzt und in einem Neutralisationsrührbehälter (4000) umgesetzt (Schritt d)). Die Temperatur beträgt dabei 115 °C und der absolute Druck 1,4 bar. Die neutralisierte Reaktionsmischung (9) wird anschließend in einem Neutralisationsabscheider (5000) in eine wässrige, untere Phase (10), die zu einem Abwassersammelbehälter (nicht gezeigt) geführt wird, und in eine organische Phase (11) getrennt (Schritt e)). Die organische, obere Phase wird zur Wäsche geleitet und in einem gerührten Waschbehälter (6000) mit Kondensat und/oder Wasser aus dem Seitenstrom der Abwasserkolonne (Anilin/Wassergemisch) (11) gewaschen (Schritt f)). Nach Abtrennung des Waschwassers (14) in einem Waschwasserabscheider (7000, Schritt g)) wird das so erhaltene Roh-MDA (13) durch Destillation in der Destillationsapparatur 6000 von Wasser und Anilin (15) befreit, wobei als Sumpfprodukt 17 t/h MDA (16) anfallen (Schritt h)). Das Waschwasser (14) wird in einen Abwassersammelbehälter (nicht gezeigt) geführt.

### Beispiel 1 (Vergleichsbeispiel): Herunterfahren der Anlage bis zum Komplettstillstand für eine Reparatur und erneute Inbetriebnahme der Anlage

Als erstes wurde die gesamte Produktionsanlage aus Beispiel 1 auf eine Produktionslast von 10 t/h MDA gebracht, um die Anlage möglichst zügig mit Anilin freispülen zu können, aber auch möglichst wenig Abfallprodukt, wie Anilin, Roh-MDA und Abwasser, die alle wieder aufbereitet werden müssen, anfallen zu lassen.

Die Abstellung der Anlage wurde mit dem Abstellen des Eingangsstromes von Formaldehyd in den Aminalreaktor begonnen. Dazu wurde die Formaldehydpumpe gestoppt und der Formaldehydweg vom Formaldehydvorratstank mit Wasser für 10 Minuten von Formaldehyd frei gespült. Nun wurde der Aminalteil der Anlage 3 Stunden mit Anilinmenge verdünnt, wobei restlicher Formaldehyd zu Aminal abreagierte und aus dem Aminalreaktor ausgespült wurde. Während des Spülvorgang wurde die Anilinmenge so erhöht, dass ein Ausgleich für die jetzt fehlende Menge an Aminal stattfand, um eine gleichbleibenden Massenstrom zu gewährleisten, und die Stände in den Folgeapparaten nicht reduzieren zu müssen. Die Reaktionswärme fiel nach Stoppen der Formalinzufuhr nicht mehr an und der Aminalreaktor kühlte sich auf 67 °C ab. Nach 3 Stunden wurde die Anilinzufuhr gestoppt, der Kühlkreislauf abgestellt und der Aminalkühler, die Aminalpumpe und der Aminalrührbehälter nacheinander in den Aminalabscheider restentleert. Der Druck im Aminalkessel verblieb während des Spülvorganges bei 1,4 bar absolut. Der Aminalabscheider wurde nun ebenfalls restentleert, indem das Spülanilin und das über dem Anilin stehende Restwasser in den ersten Umlagerungsreaktor gefahren wurden. Nun stand der Aminalteil. Das Abfahren des Aminalbereiches hat insgesamt 5 Stunden gedauert.

Als nächstes wurde die Reaktorkaskade der Umlagerungsreaktion abgefahren. Hier wurde bereits 2 Stunden nach Beginn der Abstellung des Aminalteiles der Anlage die Reaktorkaskade mit mehr Dampf beaufschlagt, um die wegbrechende Reaktionswärme zu kompensieren. Die Temperaturen in der Reaktorkaskade wurden bei 45 °C bis 165 °C belassen. Das Abfahren der Reaktorkaskade wurde mit der Beendigung der Eindosierung von der 30 %igen wässrigen Salzsäure in die Mischdüse vor dem ersten Umlagerungsreaktor zu dem Zeitpunkt, als die Restentleerung des Aminalabscheiders gestartet wurde, begonnen. Dann wurden die Reaktoren der Reaktorkaskade nacheinander zur Neutralisation leergefahren. Dampf und Vakuum wurden abgestellt, als der letzte Umlagerungstank leer war. Nun stand die Reaktorkaskade der Umlagerungsreaktion. Das Abfahren der Reaktorkaskade hat insgesamt 3 Stunden gedauert.

Danach wurde die Neutralisation außer Betrieb genommen, indem 10 Minuten länger als verdünntes Reaktionsgemisch aus der Reaktorkaskade der Umlagerungsreaktion 32 %ige Natronlauge in den Neutralisationsrührbehälter nachgefahren wurde. Dann wurde der Inhalt des Neutralisationsrührbehälters und -abscheiders in einen alkalischen Ablassbehälter restentleert. Der absolute Druck blieb bei 1,4 bar. Nun stand die Neutralisation. Der Abfahrvorgang mit Restentleerung hat 2 Stunden gedauert.

Als nächstes wurde die Wäsche außer Betrieb genommen, indem zuerst Kondensat und/oder Wasser aus dem Seitenstrom der Abwasserkolonne (Anilin/Wassergemisch) zum gerührten Waschbehälter geschlossen wurde. Der Rührer des Waschbehälters wurde abgestellt und der Inhalt des Waschbehälters in den Waschwasserabscheider entleert. Der Inhalt des Waschwasserabscheiders wurde in die Destillationsvorlage entleert. Nun stand die Wäsche. Der Abfahrvorgang hat 2 Stunden gedauert.

Als letztes wurde die Destillation abgestellt, indem nach der Restentleerung der Wäsche die komplette Destillation auf Kreislauffahrweise gestellt wurde, wobei das in der Destillation vorhandene Roh-MDA mit 6 t/h Anilin aus dem Anilinvorratstank verdünnt wurde. Der Dampf zur Destillation wurde abgestellt. Die Destillation wurde innerhalb von 4 Stunden über das noch anstehende Vakuum kalt gefahren. Im Anschluss wurde das Vakuum abgestellt und der Inhalt der kompletten Destillation (Destillationsvorlage, Wärmeaustauscher, Vordestillationskolonne mit Kondensationssystem, MDA-Kolonne mit Sumpfentnahme, Dampferzeuger) in den alkalischen Ablassbehälter entleert. Nun stand die Destillation, wobei der Abfahrvorgang 4 Stunden gedauert hat.

Während des Kaltfahrens der Destillation wurde die Abwasseraufarbeitung außer Betrieb genommen, indem zuerst das Anilin/Wassergemisch aus der Abwasserextraktion, die aus Abwassersammelbehälter, Abwassererhitzer und Anilintrennbehälter bestand, in den Abwassertank entleert wurde. Die Abwasserdestillation, bestehend aus einem Wärmeaustauscher, einer Prozessabwasserdestillationskolonne mit Kondensationssystem, einem Prozessabwasser-kühler und einer Seitenstromvorlage der Prozessabwasserdestillationskolonne, wurde abgestellt, indem der Dampf zur Abwasserdestillation abgestellt und im Anschluss der Inhalt der Abwasserdestillation in den Abwassertank entleert wurde.

Jetzt stand die komplette MDA-Anlage, die zu diesem Zeitpunkt auch komplett entleert war. Der Anlagendruck wurde auf Umgebungsdruck gestellt, indem alle Druckhaltungen der Anlage außer Betrieb genommen wurden. Die Restentleerungen aller Anlagenteile wurden nochmals geöffnet, um Reststoffe aus der Anlage abzulassen. Die Komplettabstellung mit Entleerung sämtlicher Apparate, Pumpen und Rohrleitungen hat insgesamt 24 Stunden gedauert.

**Verbrauch:** Es wurden 20 Nm³ Stickstoff zum Vakuum brechen und 500 kW Strom zur Kreislauffahrweise der Destillation verbraucht. Des Weiteren fiel ein erhöhter Dampfbedarf in der Reaktorkaskade der Umlagerungsreaktion von 5 Tonnen 6 bar Dampf und 5 Tonnen 16 bar Dampf an. Außerdem bestand ein Anfall von 10 Tonnen Spülanilin, das vor dem Einsatz in der Aminalreaktion aufbereitet werden musste.

### Durchführung einer eintägigen Instandhaltungsmaßnahme

In dem Waschbehälter musste ein defektes Rührorgan ersetzt werden.

### Vorbereitung zur Wiederinbetriebnahme der Anlage

Dabei wurden alle Anlagenteile zuerst in Kreislauffahrweise gebracht. Das Wiederanstellen der Anlage begann mit der parallelen Inbetriebnahme aller Kreisläufe der Gesamtanlage. Zuerst wurden die Anlagenteile mit Anilin und/oder Hilfsstoffen wie HCl oder NaOH befüllt.

### Befüllung des Aminalteils und Einstellung der Kreislauffahrweise:

Zuerst wurde die Anilinvorlage mit Frischanilin aus dem Anilinvorratstank befüllt. Dann wurde der leere Aminalreaktor mit Anilin befüllt, bis Anilin über das Siphon in den Aminalabscheider überlief. Als der Aminalabscheider zur Hälfte mit Anilin befüllt war, wurde der Anilinstrom zum Aminalreaktor abgestellt und die Aminalkreislauffahrweise mittels der Pumpe vom Aminalabscheider in Gang gebracht. 4 t/h Anilin wurden nun im Kreis vom Aminalabscheider über den Aminalreaktor gepumpt. Zeitbedarf: 3 Stunden.

### Befüllung der Reaktorkaskade der Umlagerungsreaktion und Einstellung der Kreislauffahrweise:

Der erste Umlagerungsreaktor wurde bis zu einem Stand von 60 % mit Frischanilin aus dem Anilinvorratstank befüllt. Dann wurde der Anilinstrom abgestellt und der erste Umlagerungsreaktor mit 24 t/h Frischanilin mittels der Austragspumpe im Kreis gepumpt. Die restlichen Umlagerungsreaktoren der Reaktorkaskade wurden aus dem sauren Ablassbehälter mit einer Mischung, die aus Anilin, Salzsäure und Spuren an Roh-MDA bestand, befüllt und die Umlagerungskreislauffahrweise mittels der Pumpen der Umlagerungsreaktoren vom letzten Umlagerungsreaktor zum zweiten Umlagerungsreaktor in Gang gebracht. 10 t/h der Mischung aus dem sauren Ablassbehälter wurden nun im Kreis gepumpt und mit Dampf auf 100 °C aufgeheizt. Die restlichen 15 Tonnen der Mischung aus dem sauren Ablassbehälter mussten später bei laufender Produktion beigemischt werden, was allerdings eine Schwankung im 2-Kerngehalt des Endproduktes bedeutet. Zeitbedarf: 8 Stunden.

### Befüllung der Neutralisation und Einstellung der Kreislauffahrweise:

2 Tonnen 32 %ige Natronlauge aus dem Natronlaugevorratstank und 8 Tonnen Kondensat aus dem Kondensatvorratsbehälter wurden in den Neutralisationsrührbehälter gefahren. Der Neutralisationsrührbehälter war nun gefüllt und 2 Tonnen der verdünnten Natronlauge über das Siphon in den Neutralisationsabscheider abgelaufen. Mittels der Pumpe des Neutralisationsabscheiders wurde die Kreislauffahrweise in Gang gebracht, indem die verdünnte Natronlauge aus dem Neutralisationsabscheider in den Neutralisationsrührbehälter gepumpt wurde. 4 t/h verdünnter Natronlauge wurden nun im Kreis vom Neutralisationsabscheider über den Neutralisationsrührbehälter gepumpt. Zeitbedarf: 4 Stunden.

### Die Wäsche wurde nicht befüllt und auch nicht in Kreislauffahrweise gestellt:

Der Rührbehälter der Wäsche und der daran angeschlossene Abscheider blieben bis zum Anfahren der Anlage leer.

### Befüllung der Destillation und Einstellung der Kreislauffahrweise:

Die Destillationsvorlage wurde mit Frischanilin aus dem Anilinvorratstank bis auf 60 % Füllstand befüllt. Aus der Destillationsvorlage wurde dann die komplette Destillation bestehend aus Wärmeaustauscher, Vordestillationskolonne mit Kondensationssystem, MDA-Kolonne mit Sumpfentnahme und Dampferzeuger mit Frischanilin befüllt, Frischanilin abgestellt und 10 t/h Frischanilin über die Vordestillationskolonne und MDA-Kolonne im Kreis gefahren. Anschließend wurde das Vakuum der Destillation in Betrieb genommen und die gesamte Destillation mit Dampf auf 100 °C erhitzt. Zeitbedarf: 5 Stunden.

### Befüllung der Abwasseraufarbeitung und Einstellung der Kreislauffahrweise:

Aus dem Abwassertank wurde Abwasser in den Abwassersammelbehälter gepumpt. Aus dem Abwassersammelbehälter wurde nun Abwasser in den Abwassererhitzer und Anilintrennbehälter gefördert. Frischanilin aus dem Anilinvorratstank wurde nun auf den Abwassererhitzer gegeben, der Abwassererhitzer anschließend auf 90 °C aufgeheizt und das Gemisch aus Frischanilin und Abwasser aus dem Abwassererhitzer über den Anilintrennbehälter und den Abwassersammelbehälter im Kreis gefahren. Die Abwasserdestillation blieb bis zum Anfahren der Anlage außer Betrieb. Zeitbedarf: 5 Stunden.

Insgesamt wurden 15 Stunden benötigt, um die Gesamtanlage, wie beschrieben, in Kreislauffahrweise zu versetzen, weil Teile der Anlage parallel befüllt wurden. Dazu wurden 50 Tonnen 16 bar Dampf und 9500 kW an Strom zum Betreiben der Motoren benötigt.

### Wiederinbetriebnahme der Anlage

Die Anlage lief, wie in den Vorbereitungen zur Wiederinbetriebnahme der Anlage beschrieben, in den einzelnen Betriebssegmenten im Kreis, d.h. sie war aufgeheizt, Rührer waren in Betrieb, in den benötigten Bereichen lag Beschleierungsdruck mit Stickstoff und Vakuum an. Einsatzstoffe und Hilfsstoffe standen bereit.

### Anfahren der Destillation mit Vakuumsystem:

Die Destillation mit Vakuumsystem stand in Kreislauffahrweise. Das Vakuumsystem der Vordestillationskolonne und MDA-Kolonne wurde in Betrieb genommen und auf 120 mbar absolut eingestellt. Dann wurde der 16 bar Dampf (Verbrauch: 40 Tonnen) zur Vordestillationskolonne und der 110 bar Dampf (Verbrauch: 10 Tonnen) zur MDA-Kolonne geöffnet und die Kolonnen aufgeheizt. Die Temperatur in der Vordestillationskolonne betrug 190 °C und in der MDA-Kolonne 225 °C. Das zum Destillieren notwendige Anilin wurde für die Zeit der Kreislauffahrweise aus der Anilinvorlage in die Pumpenvorlage der Destillation eingespeist. Der Dampferzeuger war in Betrieb. Nun war das Betriebssegment der Destillation bereit, Roh-MDA aufzunehmen. Zeitbedarf: 3 Stunden.

### Anfahren der Aminalreaktion:

30 Minuten bevor die Destillation bereit war, Roh-MDA aufzunehmen, wurde die Aminalherstellung gestartet, indem Anilin zum Aminalreaktor geöffnet und 10 Minuten später der Formalinstrom angestellt wurde. Gleichzeitig wurde der Weg der 90 °C heißen organischen Phase aus dem Aminalabscheider zum ersten Reaktor der Umlagerungsreaktion geöffnet und die Temperatur im ersten Umlagerungsreaktor wurde mittels Vakuum auf 50 °C abgesenkt. Jetzt konnte die saure Katalyse der Umlagerungsreaktion mit Salzsäure gestartet werden. Das im Aminalabscheider anfallende Aminalwasser wurde der Abwasseraufarbeitung zugeführt. Nun war das Betriebssegment der Aminalreaktion angestellt und Aminallösung ging zur Umlagerungsreaktion. Zeitbedarf: 15 Minuten.

### Anfahren der Umlagerungsreaktion:

Nachdem der Salzsäurestrom in Betrieb genommen wurde und die Temperatur im ersten Umlagerungsreaktor stand, wurden die weiteren Umlagerungsreaktoren und Verweilzeittürme der Reaktorkaskade auf 60 °C bis zum letzten Reaktor auf 165 °C aufgeheizt (Verbrauch: 60 Tonnen 16 bar Dampf). Nun war das Betriebssegment der Umlagerungsreaktion angestellt und die Kondensationslösung, bestehend aus MDA, Anilin und Salzsäure (Roh-MDA) wurde als nächstes neutralisiert. Zeitbedarf: 10 Minuten.

### Anfahren der Neutralisation:

Die Natronlaugedosiereinrichtung wurde in Betrieb genommen, indem Natronlauge und Waschwasser zum Neutralisationsrührbehälter gefahren wurden. 10 Minuten später wurde der Weg der sauren Kondensationslösung aus der Umlagerungsreaktion aufgeschaltet. Nun war das Betriebssegment der Neutralisation angestellt und das neutralisierte Roh-MDA konnte gewaschen werden. Zeitbedarf: 10 Minuten.

### Anfahren der Wäsche:

116 °C heißes, neutralisiertes Roh-MDA kam im MDA-Wäscher an und wurde mit Kondensat gewaschen. Die Waschwasserzugabe, bestehend aus Kondensat und/oder dem Seitenstrom der Prozessabwasserkolonne, wurde angestellt. Nun war das Betriebssegment der Wäsche angestellt. Neutralisiertes und gewaschenes Roh-MDA verließ den Phasentrennapparat und ging zur Destillation. Zeitbedarf: 5 Minuten.

### Anfahren der Abwasseraufarbeitung:

Sobald die Neutralisation und Wäsche liefen, wurde die Abwasseraufarbeitung angestellt, indem die Abwasserextraktion und die Abwasserdestillation in Betrieb genommen wurden. Dazu wurde das anfallende Abwasser aus den vorbeschriebenen Verfahrensschritten (Neutralisation, Wäsche und Destillation), das im Abwassersammelbehälter ankam, mittels Pumpe über den Prozessabwassererhitzer in den Anilintrennbehälter gefahren. Von dort aus ging das extrahierte Abwasser in die Abwasserdestillation. Die Abwasserdestillation wurde mit 20 Tonnen 6 bar Dampf auf 107 °C erhitzt, und das Abwasser verließ die Produktionsanlage. Zeitbedarf: 2 Stunden.

Die komplette MDA-Anlage lief jetzt mit einer reduzierten Last von 10 t/h MDA und konnte nun auf die gewünschte Sollproduktion hochgefahren werden. Insgesamt wurden 10 Stunden benötigt, um die Gesamtanlage, wie beschrieben, aus der Kreislauffahrweise in Betrieb zu nehmen und erstes Endprodukt in den MDA-Tank auszuschleusen. Dazu wurden 100 Tonnen 16 bar Dampf, 10 Tonnen 110 bar Dampf und 20 Tonnen 6 bar Dampf sowie 6315 kW Strom zum Betreiben der Motoren benötigt.

Es war dabei zwingend erforderlich, die Produktionsanlage mit reduzierter Last anzufahren, da ansonsten die benötigten Temperaturprofile für Aminal- und Umlagerungsreaktion, Neutralisation, Wäsche und Abwasseraufarbeitung und Destillation nicht schnell genug zur Verfügung stehen. Dieses würde zu unvollständigen Reaktionen, vermehrten Nebenprodukten und mangelhafter Aufarbeitung des Produktes führen.

### Fazit:

Der Zeitaufwand für den gesamten Produktionsstillstand (Abfahren, Maßnahme und Anfahren) umfasste 73 Stunden.

Der Energieverbrauch dafür (Abfahren, Maßnahme und Anfahren) betrug 15815 kW Strom, 105 Tonnen 16 bar Dampf, 10 Tonnen 110 bar Dampf und 25 Tonnen 6 bar Dampf. Darüber hinaus wurden Hilfsstoffe in Form von 20 Nm³ Stickstoff zum Brechen des Vakuums verbraucht.

### Beispiel 2 (erfindungsgemäß): Anlage in Kreislauffahrweise bringen, Reparatur in der Wäsche, Anlage aus der Kreislauffahrweise heraus wieder anfahren.

Als erstes wurde die gesamte Produktionsanlage wie im Vergleichsbeispiel 1 auf die optimale Produktionslast von 10 t/h MDA gebracht, um danach die gesamte Anlage in Kreislauffahrweise zu bringen.

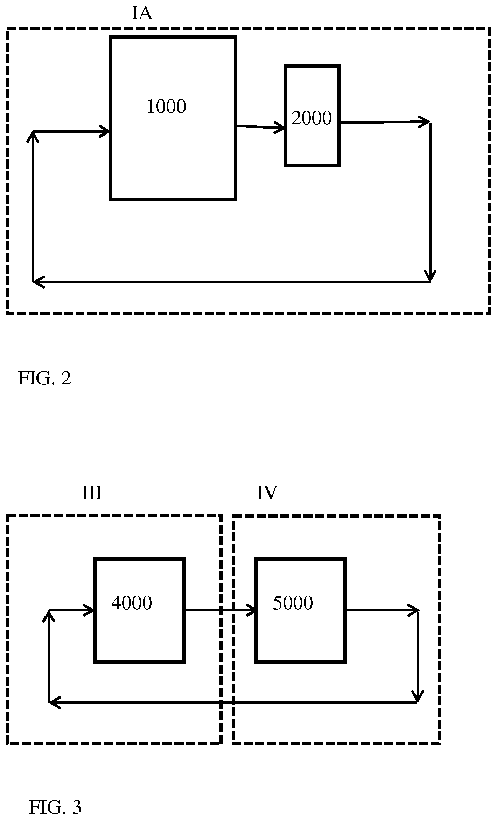

Die eigentliche Einstellung der Anlage auf Kreislauffahrweise begann mit dem Abstellen des Eingangsstromes von Formaldehyd in den Aminalreaktor. Dazu wurde die Formaldehydpumpe gestoppt und der Formaldehydweg vom Formaldehydvorratstank mit Wasser für 10 Minuten von Formaldehyd frei gespült. Nun wurde der Aminalteil der Anlage 30 Minuten mit Anilin verdünnt, wobei Formaldehyd weiterhin zu Aminal abreagierte und die Aminallösung verdünnt wurde. Während des Spülvorganges wurde die Anilinmenge so erhöht, dass ein Ausgleich für die jetzt fehlende Menge an Aminal stattfand, um einen gleichbleibenden Massenstrom zu gewährleisten und um die Stände in den Folgeapparaten nicht reduzieren zu müssen. Die Reaktionswärme fiel nach Stoppen der Formaldehydzufuhr nicht mehr an und der Aminalreaktor kühlte sich auf 67 °C ab. Nach 30 Minuten wurde die Anilinzufuhr gestoppt und der Aminalteil der Anlage auf Kreislauffahrweise gestellt, indem das mit Anilin verdünnte Aminal ungekühlt vom Aminalreaktor über das Siphon in den Aminalabscheider und von dort im Kreis zurück zum Aminalreaktor gepumpt wurde (FIG. 2). Der Druck im Aminalkessel verblieb während der Kreislauffahrweise bei 1,4 bar absolut. Das Einstellen des Aminalteiles der Anlage in Kreislauffahrweise dauerte insgesamt 1 Stunde.

Als nächstes wurde die Reaktorkaskade der Umlagerungsreaktion in Kreislauffahrweise gebracht, indem zunächst der Salzsäurestrom und dann der Aminalstrom abgestellt wurden. Die Kondensationslösung, bestehend aus MDA, Anilin und Salzsäure konnte dann ohne Beheizung vom letzten Umlagerungsreaktor in den ersten Umlagerungsreaktor und über die Reaktorkaskade im Kreis gepumpt werden. Das Einstellen des Umlagerungsteiles der Anlage auf Kreislauffahrweise dauerte insgesamt 1 Stunde.

Als nächstes wurde die Neutralisation in Kreislauffahrweise gestellt, indem zuerst die saure Kondensationslösung, die aus der Umlagerungsreaktion kam, und 10 Minuten später die 32%ige Natronlauge und das Waschwasser abgestellt wurden. Dann wurde der Inhalt des Neutralisationsabscheiders mittels der Kreislaufpumpe aus dem Neutralisationsabscheider in den Neutralisationsrührbehälter über das Siphon zurück in den Neutralisationsabscheider gepumpt. Damit stand die Kreislauffahrweise (FIG. 3). Der Druck in der Neutralisation blieb bei 1,4 bar absolut. Das Einstellen des Neutralisationsteiles der Anlage auf Kreislauffahrweise dauerte insgesamt 40 Minuten.

Als nächstes wurde die Wäsche abgestellt, indem die Waschwasserzugabe, bestehend aus Kondensat, zum gerührten Waschbehälter geschlossen wurde. Der Rührer des Waschbehälters wurde abgestellt. Um die Reparaturmaßnahme in der Wäsche vorzubereiten, wurde der Inhalt des Waschbehälters in den Waschwasserabscheider entleert. Der Inhalt des Waschwasserabscheiders wurde in die Destillationsvorlage entleert. Nun stand die Wäsche. Dieser Abfahrvorgang dauerte 2 Stunden.

Als letztes wurde die Destillation auf Kreislauffahrweise gestellt, indem nach der Restentleerung der Wäsche das in der Destillation vorhandene Roh-MDA mit 6 t/h Anilin aus dem Anilinvorratstank verdünnt wurde. Es kam kein Roh-MDA in der Pumpenvorlage der Destillation mehr an. Der Sumpfablauf der MDA-Kolonne wurde über den Dampferzeuger und den Wärmeaustauscher zurück auf die Pumpenvorlage der Destillation gestellt und somit über die Pumpenvorlage, den Wärmeaustauscher, die Vordestillationskolonne und zurück in den Sumpf der MDA-Kolonne im Kreis gefahren. Nun konnte der Dampf zur Vordestillationskolonne und MDA-Kolonne abgestellt werden. Anschließend konnte das Vakuumsystem der beiden Kolonnen abgestellt werden. Das Einstellen des Destillationsteiles der Anlage auf Kreislauffahrweise dauerte insgesamt 3 Stunden.

Zuletzt wurde die Abwasseraufarbeitung auf Kreislauffahrweise genommen, als kein Prozesswasser mehr anfiel. Die Abwasserextraktion, bestehend aus Abwassersammelbehälter, Abwassererhitzer und Anilintrennbehälter, wurde auf Kreislauffahrweise gestellt, indem der Ablauf des Anilintrennbehälters auf den Abwassersammelbehälter gestellt und mittels Pumpe über den Prozessabwassererhitzer in den Anilintrennbehälter im Kreis gepumpt wurde. Die Kreislauffahrweise konnte ohne Beheizung auf unbestimmte Zeit betrieben werden. Die Abwasserdestillation, bestehend aus einem Wärmeaustauscher, einer Prozessabwasserdestillationskolonne mit Kondensationssystem, einem Prozessabwasserkühler und einer Seitenstromvorlage der Prozessabwasserdestillationskolonne, wurde abgestellt, indem der Dampf zur Kolonne abgestellt wurde. Eine Kreislauffahrweise der Abwasserdestillation war hierbei nicht vorgesehen.

Jetzt lief die komplette MDA-Anlage bis auf die Wäsche in Kreislauffahrweise. Das Einstellen der Kreislauffahrweise hatte 6 Stunden in Anspruch genommen.

**Verbrauch:** 20 Nm³ Stickstoff zum Vakuum brechen, und 3825 kW Strom um die Anlage in Kreislauffahrweise zu bringen, Anfall von 10 Tonnen Spülanilin in der Destillation, das vor dem Einsatz in der Aminalreaktion aufbereitet werden musste.

### Durchführung einer eintägigen Instandhaltungsmaßnahme

An dem Waschbehälter musste ein defektes Schauglas und eine leckende Dichtung gewechselt werden. Für die Kreislauffahrweise während der Maßnahme wurden 15300 kW an Strom benötigt. Es wurde lediglich ein wenig Dampf verbraucht, um die Kreisläufe auf Temperatur zu halten (12 Tonnen 16 bar Dampf).

### Vorbereitung zur Wiederinbetriebnahme der Anlage

Die Vorbereitungen zur Wiederinbetriebnahme der Anlage entfielen, da schon alle Anlagenteile in Kreislauffahrweise liefen. Daher entfiel auch das Befüllen der Anlagenteile mit Anilin und/oder Hilfsstoffen wie Salzsäure oder Natronlauge.

### Wiederinbetriebnahme der Anlage

Die Anlage lief, wie zuvor in den Vorbereitungen zur Wiederinbetriebnahme der Anlage beschrieben, in den einzelnen Betriebssegmenten im Kreis. Einsatzstoffe und Hilfsstoffe standen bereit, die Anlagenteile waren aufgeheizt, Rührer waren in Betrieb, in den benötigten Bereichen lag Beschleierungsdruck mit Stickstoff an, und Vakuum lag ebenso an.

Die Wiederinbetriebnahme der Anlage wurde, wie im Beispiel 1 (Vergleichsbeispiel) beschrieben, durchgeführt. Die komplette MDA-Anlage lief jetzt mit einer reduzierten Last von 10 t/h MDA und konnte nun auf die gewünschte Sollproduktion hochgefahren werden. Insgesamt wurden wieder 10 Stunden benötigt, um die Gesamtanlage, wie beschrieben, aus der Kreislauffahrweise in Betrieb zu nehmen und erstes Endprodukt in den MDA-Tank auszuschleusen. Dazu wurden ebenfalls 100 Tonnen 16 bar Dampf, 10 Tonnen 110 bar Dampf und 20 Tonnen 6 bar Dampf sowie 6315 kW Strom zum Betreiben der Motoren benötigt.

Der Zeitaufwand für die gesamte Aktion (Abfahren, Ausführen der Maßnahme und Anfahren) betrug 40 Stunden. Somit ergab sich bei einer Nennlast von 380 Tagestonnen eine Mehrproduktion von 522,5 Tonnen MDA im Vergleich zu Beispiel 1 (Vergleichsbeispiel).

Der Energieverbrauch für die gesamte Aktion (Abfahren, Maßnahme und Anfahren) umfasste 25500 kW Strom, 112 Tonnen 16 bar Dampf, 10 Tonnen 110 bar Dampf und 25 Tonnen 6 bar Dampf sowie einen Verbrauch von Hilfsstoffen in Form von 20 Nm³ Stickstoff zum Vakuum brechen.

**Fazit:** es werden im erfindungsgemäßen Beispiel 2 mit Kreislauffahrweise 7 Tonnen 16 bar Dampf und 9685 kW Strom mehr verbraucht als bei einer Komplettabstellung der Anlage wie in Beispiel 1 (Vergleichsbeispiel). Dafür ergibt sich aber eine stark verbesserte Produktivität der Anlage, da wegen des geringeren Zeitbedarfes für die ganze Aktion (Abfahren, Maßnahme und Anfahren) über 500 Tonnen an MDA mehr produziert werden konnten.

## Patentansprüche

1. Verfahren zum Betrieb einer Anlage zur Herstellung von Di- und/oder Polyaminen der Diphenylmethanreihe, wobei das Verfahren einen Produktionsstillstand umfasst und die Anlage die folgenden Anlagenteile umfasst:
IA) einen Reaktor mit integrierter Phasentrenneinrichtung oder einen Reaktor und einen separaten Phasentrennapparat zur Reaktion von Anilin und Formaldehyd in Abwesenheit eines sauren Katalysators unter Bildung eines Aminals und anschließender Trennung des erhaltenen Reaktionsgemisches in eine wässrige und eine organische, das Aminal enthaltenden Phase und
IIA) einen Reaktor zur Reaktion der im Reaktor IA) erhaltenen organischen, das Aminal enthaltenden Phase mit Säure,
oder
IB) einen Reaktor zur Reaktion von Anilin mit Säure und
IIB) einen Reaktor zur Reaktion des im Reaktor IB) erhaltenen Reaktionsgemisches mit Formaldehyd;
und
III) einen Reaktor zur Neutralisation des Reaktionsgemisches aus IIA) oder IIB);
IV) einen Trennbehälter zum Auftrennen des neutralisierten Reaktionsgemisches aus III) in eine organische Phase, umfassend Di- und Polyamine der Diphenylmethanreihe und eine wässrige Phase;
V) einen Waschbehälter zum Waschen der organischen Phase aus IV) mit Waschflüssigkeit;
VI) einen Trennbehälter zum Auftrennen des Gemisches aus V) in eine organische Phase, umfassend Di- und Polyamine der Diphenylmethanreihe und eine wässrige Phase;
VII) eine Destillationseinrichtung zum Destillieren der organischen Phase aus VI) unter Erhalt von Di- und/oder Polyaminen der Diphenylmethanreihe und eines Wasser und Anilin enthaltenden Stroms;
sowie optional
VIII) eine Abwasseraufarbeitungseinrichtung zur Aufarbeitung der wässrigen Phase aus IA) und/oder der wässrigen Phase aus IV) und/oder der wässrigen Phase aus VI) und/oder des Wasser und Anilin enthaltenden Stroms aus VII), bevorzugt umfassend einen Abwassersammelbehälter, Abwassererhitzer und Anilintrennbehälter;
**dadurch gekennzeichnet, dass**
zur Durchführung des Produktionsstillstandes nicht die gesamte Anlage außer Betrieb genommen wird, sondern nur einzelne Anlagenteile außer Betrieb genommen werden, wobei zur Außerbetriebnahme dieser Anlagenteile die folgenden Schritte durchlaufen werden:
(i) a) Stoppen der Zufuhr von Formaldehyd in den Reaktor IA);
(ii) a) Stoppen der Zufuhr von Anilin in den Reaktor IA);
(iii) a) Stoppen der Zufuhr von Säure in den Reaktor IIA);
oder
(i) b) Stoppen der Zufuhr von Formaldehyd in den Reaktor IIB);
(ii) b) Stoppen der Zufuhr von Säure in den Reaktor IB);
(iii) b) Stoppen der Zufuhr von Anilin in den Reaktor IB);
und
(iv) Betreiben, wobei gegebenenfalls der Waschbehälter V) und der Trennbehälter VI) ausgenommen werden, aller Anlagenteile, die nicht vom Produktionsstillstand betroffen sind, in Kreislauffahrweise durch Verwenden des Ausgangstroms eines Anlagenteils als Eingangsstrom dieses Anlagenteils oder eines anderen Anlagenteils, welcher dem betreffenden Anlagenteil vorgeschaltet ist;
(v) Außerbetriebnahme mindestens eines Anlagenteils;
(vi) Optional Öffnen des in Schritt (v) außer Betrieb genommenen mindestens einen Anlagenteils;
(vii) Durchführen einer Instandhaltungs-, Reinigungs- und/oder Reparaturmaßnahme in dem mindestens einen in Schritt (v) außer Betrieb genommenen Anlagenteil;
(viii) Optional Schließen und optional Inertisieren des mindestens einen in Schritt (v) außer Betrieb genommenen Anlagenteils.

2. Verfahren gemäß Anspruch 1, bei dem die Anlage das Anlagenteil VIII) umfasst.

3. Verfahren gemäß einem der Ansprüche 1 oder 2, bei dem in Schritt (iv) alle Anlagenteile, die nicht vom Produktionsstillstand betroffen sind, in Kreislauffahrweise gebracht werden.

4. Verfahren gemäß einem der Ansprüche 1 oder 2, bei dem in Schritt (iv) die Anlagenteile Waschbehälter V) und Trennbehälter VI) von der Kreislauffahrweise ausgenommen werden.

5. Verfahren gemäß mindestens einem der Ansprüche 1 bis 4, wobei zur Wiederaufnahme des Produktionsverfahrens zur Herstellung der Di- und Polyamine der Diphenylmethanreihe im Anschluss an Schritt (viii) alle Anlagenteile in einer Reihenfolge wieder in Betrieb genommen werden, die der Reihenfolge der Außerbetriebnahme bzw. des Umstellens auf Rückführung des Ausgangsstroms des jeweiligen Anlagenteils als Eingangsstrom des jeweiligen Anlagenteils entgegengesetzt ist.

## Claims

1. Method of operating a plant for preparing diamines and/or polyamines of the diphenylmethane series, where the method includes a production stoppage and the plant comprises the following plant parts:
IA) a reactor having an integrated phase separation facility or a reactor and a separate phase separation apparatus for the reaction of aniline and formaldehyde in the absence of an acid catalyst to form an aminal and subsequently separation of the reaction mixture obtained into an aqueous phase and an organic phase containing the aminal and
IIA) a reactor for reaction of the organic phase containing the aminal obtained in the reactor IA) with acid;
or
IB) a reactor for the reaction of aniline with acid and
IIB) a reactor for reaction of the reaction mixture obtained in the reactor IB) with formaldehyde;
and
III) a reactor for neutralization of the reaction mixture from IIA) or IIB);
IV) a separation vessel for separation of the neutralized reaction mixture from III) into an organic phase comprising diamines and polyamines of the diphenylmethane series and an aqueous phase;
V) a washing vessel for washing of the organic phase from IV) by means of washing liquid;
VI) a separation vessel for separation of the mixture from V) into an organic phase comprising diamines and polyamines of the diphenylmethane series and an aqueous phase;
VII) a distillation apparatus for distillation of the organic phase from VI) to give diamines and/or polyamines of the diphenylmethane series and a stream containing water and aniline;
and optionally
VIII) a wastewater work-up facility for work-up of the aqueous phase from IA) and/or the aqueous phase from IV) and/or the aqueous phase from VI) and/or the stream containing water and aniline from VII), preferably comprising a wastewater collection vessel, wastewater heater and aniline separation vessel;
**characterized in that**
the production stoppage is implemented not by shutting down the entire plant but by shutting down only individual plant parts, where the shutdown of these plant parts is implemented by carrying out the following steps:
(i) a) stopping of the introduction of formaldehyde into the reactor IA);
(ii) a) stopping of the introduction of aniline into the reactor IA);
(iii) a) stopping of the introduction of acid into the reactor IIA);
or
(i) b) stopping of the introduction of formaldehyde into the reactor IIB);
(ii) b) stopping of the introduction of acid into the reactor IB);
(iii) b) stopping of the introduction of aniline into the reactor IB);
and
(iv) operation, optionally with exclusion of the washing vessel V) and the separation vessel VI), of all plant parts unaffected by the production stoppage in circulation mode by using the output stream from a plant part as feed stream to that plant part or another plant part upstream of the plant part in question;
(v) shutdown of at least one plant part;
(vi) optionally opening of the at least one plant part which has been shut down in step (v);
(vii) performing of a maintenance, cleaning and/or repair measure in the at least one plant part which has been shut down in step (v);
(viii) optionally closing and optionally making inert of the at least one plant part which has been shut down in step (v).

2. Method according to Claim 1, in which the plant includes plant part VIII).

3. Method according to either of Claims 1 and 2, in which, in step (iv), all plant parts unaffected by the production stoppage are put into circulation mode.

4. Method according to either of Claims 1 and 2, in which, in step (iv), the plant parts of washing vessel V) and separation vessel VI) are excluded from the circulation mode.

5. Method according to any of Claims 1 to 4, wherein, to restart the production process for preparing the diamines and polyamines of the diphenylmethane series, all plant parts are started up again after step (viii) in an order which is the reverse of the order of the shutdown or of the changing to recirculation of the output stream from the respective plant part as feed stream to the respective plant part.

## Revendications

1. Procédé pour le fonctionnement d'une installation pour la préparation de diamines et/ou de polyamines de la série du diphénylméthane, le procédé comprenant un arrêt de production et l'installation comprenant les parties d'installation suivantes :
IA) un réacteur comportant un dispositif de séparation de phases intégré ou un réacteur et un appareil de séparation de phases séparé pour la réaction d'aniline et de formaldéhyde en absence d'un catalyseur acide avec formation d'un aminal et séparation subséquente du mélange réactionnel obtenu en une phase aqueuse et une phase organique contenant l'aminal et
IIA) un réacteur pour la réaction de la phase organique contenant l'aminal obtenue dans le réacteur IA) avec un acide,
ou
IB) un réacteur pour la réaction d'aniline avec un acide et
IIB) un réacteur pour la réaction du mélange réactionnel obtenu dans le réacteur IB) avec du formaldéhyde ;
et
III) un réacteur pour la neutralisation du mélange réactionnel de IIA) ou de IIB) ;
IV) un récipient de séparation pour la séparation du mélange réactionnel neutralisé de III) en une phase organique, comprenant des diamines et des polyamines de la série du diphénylméthane et une phase aqueuse ;
V) un récipient de lavage pour le lavage de la phase organique de IV) avec un liquide de lavage ;
VI) un récipient de séparation pour la séparation du mélange de V) en une phase organique, comprenant des diamines et des polyamines de la série du diphénylméthane et une phase aqueuse ;
VII) un dispositif de distillation pour la distillation de la phase organique de VI) avec obtention de diamines et/ou de polyamines de la série du diphénylméthane et d'un flux contenant de l'eau et de l'aniline ;
ainsi qu'éventuellement
VIII) un dispositif de traitement des eaux usées pour le traitement de la phase aqueuse de IA) et/ou de la phase aqueuse de IV) et/ou de la phase aqueuse de VI) et/ou du flux contenant de l'eau et de l'aniline de VII), préférablement comprenant un récipient de collecte d'eaux usées, un réchauffeur d'eaux usées et un récipient de séparation d'aniline ;
**caractérisé en ce que**
pour la réalisation de l'arrêt de production, l'installation entière n'est pas mise hors service, mais seulement des parties d'installation individuelles sont mises hors service, dans lequel pour la mise hors service de ces parties d'installation, les étapes suivantes sont réalisées
(i)(a) arrêt de l'alimentation de formaldéhyde dans le réacteur IA) ;
(ii)(a) arrêt de l'alimentation d'aniline dans le réacteur IA) ;
(iii)(a) arrêt de l'alimentation d'acide dans le réacteur IIA) ;
ou
(i)(b) arrêt de l'alimentation de formaldéhyde dans le réacteur IIB) ;
(ii)(b) arrêt de l'alimentation d'acide dans le réacteur IB) ;
(iii)(b) arrêt de l'alimentation d'aniline dans le réacteur IB) ;
et
(iv) fonctionnement de toutes les parties d'installation qui ne sont pas concernées par l'arrêt de production, éventuellement le récipient de lavage V) et le récipient de séparation VI) étant éventuellement exclus, en mode de fonctionnement circulant par l'utilisation du flux de sortie d'une partie d'installation en tant que flux d'entrée de cette partie d'installation ou d'une autre partie d'installation, qui est placée en amont de la partie d'installation concernée ;
(v) mise hors service d'au moins une partie d'installation ;
(vi) ouverture éventuelle de l'au moins une partie d'installation mise hors service dans l'étape (v) ;
(vii) réalisation d'une mesure de maintenance, de nettoyage et/ou de réparation dans l'au moins une partie d'installation mise hors service dans l'étape (v) ;
(viii) fermeture éventuelle et inertisation éventuelle de l'au moins une partie d'installation mise hors service dans l'étape (v) .

2. Procédé selon la revendication 1, dans lequel l'installation comprend la partie d'installation VIII) .

3. Procédé selon l'une quelconque des revendications 1 et 2, dans lequel dans l'étape (iv) toutes les parties installation qui ne sont pas concernées par l'arrêt de production sont amenées en mode de fonctionnement circulant.

4. Procédé selon l'une quelconque des revendications 1 et 2, dans lequel dans l'étape (iv) les parties d'installation : récipient de lavage V) et récipient de séparation VI) sont exclues du mode de fonctionnement circulant.

5. Procédé selon au moins l'une des revendications 1 à 4, dans lequel pour la reprise du procédé de production pour la préparation de diamines et/ou de polyamines de la série du diphénylméthane, à la suite de l'étape (viii) toutes les parties d'installation reprennent un fonctionnement dans un ordre qui est contraire à l'ordre de la mise hors service respectivement de la conversion au recyclage du flux de sortie de la partie d'installation respective en tant que flux d'entrée de la partie d'installation respective.
